# EUROPÄISCHE PATENTSCHRIFT

(11) **EP 1 534 848 B1**
(45) Veröffentlichungstag und Bekanntmachung des Hinweises auf die Patenterteilung: **22.08.2007**
(21) Anmeldenummer: 03790885.2
(22) Anmeldetag: 08.08.2003
(51) Int. Cl.: C12P 1/00, C12P 21/00

(54) **VERFAHREN ZUR EXTRAKTION VON PROTEINEN**
METHOD FOR EXTRACTING PROTEINS
PROCEDE POUR EXTRAIRE DES PROTEINES

(30) Priorität: 02.09.2002 EP 02019570
(43) Veröffentlichungstag der Anmeldung: 01.06.2005
(73) Patentinhaber: Merck Patent GmbH, 64293 Darmstadt (DE)
(72) Erfinder: HENDRICKS, Robertus, 69121 Heidelberg (DE); ABDOLZADE-BAVIL, Afsaneh, 69214 Eppelheim (DE); ANDERS, Jonas, 64293 Darmstadt (DE)
(86) Internationale Anmeldenummer: PCT/EP2003/008806
(87) Internationale Veröffentlichungsnummer: WO 2004/020646

(56) Entgegenhaltungen:
- SINIBALDI R M ET AL: "PUTATIVE FUNCTION OF DROSOPHILA-MELANOGASTER HEAT SHOCK PROTEINS IN THE NUCLEOSKELETON" JOURNAL OF BIOLOGICAL CHEMISTRY, Bd. 256, Nr. 21, 1981, Seiten 10735-10738, XP002266600 ISSN: 0021-9258
- FEY E G ET AL: "EPITHELIAL CYTOSKELETAL FRAMEWORK AND NUCLEAR MATRIX INTERMEDIATE FILAMENT SCAFFOLD 3 DIMENSIONAL ORGANIZATION AND PROTEIN COMPOSITION" JOURNAL OF CELL BIOLOGY, Bd. 98, Nr. 6, 1984, Seiten 1973-1984, XP002266743 ISSN: 0021-9525
- RAMSBY M L ET AL: "DIFFERENTIAL DETERGENT FRACTIONATION OF ISOLATED HEPATOCYTES: BIOCHEMICAL, IMMUNOCHEMICAL AND TWO-DIMENSIONAL GEL ELECTROPHORESIS CHARACTERIZATION OF CYTOSKELETAL AND NONCYTOSKELETAL COMPARTMENTS" ELECTROPHORESIS, WEINHEIM, DE, Bd. 15, Nr. 2, 1994, Seiten 265-277, XP008025972 ISSN: 0173-0835 in der Anmeldung erwähnt

## Beschreibung

Die Erfindung betrifft ein Verfahren zur Extraktion zellulärer Proteine in Abhängigkeit ihrer subzellulären Lokalisierung (Topologie). Insbesondere ermöglicht das erfindungsgemäße Verfahren durch die Aufteilung des Gesamtproteoms in Partialproteome die Isolierung von Proteinen des Zellkerninneren als eigenständige Fraktion und macht diese Fraktion damit erstmals in ausreichender Reinheit und Qualität für weitere Analysen zugänglich.

Mit der Sequenzierung des menschlichen Genoms erhält die Wissenschaft Zugang zum individuellen genetischen Code eines jeden Menschen. Daraus ergeben sich Informationen über seine Abstammung und Herkunft. Für die Untersuchung der biologischen Funktion einzelner Gene bzw. der entsprechenden Proteine ist diese Information aber nicht ausreichend. Das komplexe Netzwerk einer Zelle lässt sich nicht einfach durch das Entschlüsseln der genomischen DNA eines Menschen charakterisieren. Eine Untersuchung der Proteine, die vom Genom kodiert werden, muss sich der Genomanalyse anschließen, da nur mit dieser Zusatzinformation das dynamische Geschehen des menschlichen Organismus auf molekularer Ebene beschreibbar ist. Auch gibt es oftmals nur eine geringe Korrelation zwischen der Gen-Transkription und dem entsprechenden Translationsprodukt, so dass nur mit Hilfe der Proteomanalyse erkennbar wird, welche Proteine unter gegebenen Einflüssen wie stark exprimiert und eventuell posttranslational verändert werden (Allen, L.: Functional Genomics Nature 405 (2000) 819-865; Ezzell, C.: The Business of the Human Genome Scientific American July (2000) 39-57).

Um Proteine z.B. bezüglich ihres Expressionsmusters oder ihrer Funktion untersuchen zu können, müssen diese zuerst für die entsprechenden Analysen zugänglich gemacht werden.

Die parallele Auftrennung und Analyse aller Proteine einer Zelle ist mit den heutigen Methoden nahezu unmöglich. Eine humane Zelle exprimiert zwischen 35.000 und 50.000 Proteine, die mit einem Faktor 10⁶ in der Abundanz variieren. Auch mit der momentan höchst auflösenden Protein-Trenntechnik, der 2 dimensionalen Gelelektrophorese, lassen sich maximal 5.000 bis 10.000 Proteine mit hoher Auflösung separieren, wobei aus einem Gesamtlysat hauptsächlich die Proteine mit hoher Expressionsrate visualisiert werden können. Da aber in den meisten Fällen eher niedrigabundante Proteine in pathologischen Prozessen involviert sind, ist unter klinischen und diagnostischen Gesichtspunkten vor der Proteinanalyse eine Fraktionierung der Proteine einer Zelle in Untereinheiten, also die Herstellung von Partialproteomen, erforderlich.

Zur Klärung der Funktion eines Proteins sind verschiedene Basistechniken wie z.B. Aktivitäts-Assays oder Proteinwechselwirkungsuntersuchungen entwickelt worden. Diese setzten allerdings zumeist voraus, dass die zu untersuchenden Proteinen der jeweiligen Funktionsanalyse in nativem, nicht denaturierten Zustand zugeführt werden.

Ein wertvoller ergänzender Ansatz zur Untersuchung der Funktion von Proteinen ist die Analyse der subzellulären Lokalisierung von Proteinen oder deren Umverteilung in einer Zelle. Viele Regelungsvorgänge in einer Zelle führen nach Wechselwirkung mit einem Signalmolekül zur Änderung der subzelluläre Lokalisierung, wobei z.B. durch Übergang eines Proteins vom Cytosol in den Zellkern eine Änderungen der RNA-Transkription hervorgerufen wird.

Somit steigt die Nachfrage nach standardisierten Verfahren zur Herstellung von Partialproteomen, welche zum einen eine Reduktion der Gesamtzahl der parallel zu untersuchenden Proteine bewirken und es zum anderen erlauben, die subzelluläre Lokalisierung (Topologie) als zusätzliche Information in die funktionelle Proteomanalyse einzubeziehen.

Es sind verschiedene Methoden zur Herstellung von Partialproteomen bekannt, die jedoch durch die Art der Aufreinigung jeweils nur einen Teil des Proteoms für die Analyse zugänglich machen. Beispiele derartiger Methoden sind die Aufreinigung vereinzelter Zellkompartimente basiered entweder auf unterschiedlicher Netto-Ladung oder unterschiedlicher Dichte der Kompartimente, auf unterschiedlicher Proteinzusammensetzung, die für Affinitätsaufreinigung genutzt werden kann, oder auf unterschiedlicher Löslichkeit der Organellproteine in Gegenwart von speziellen Puffern.

Da bei diesen Verfahren nur ein Teil des Gesamtproteoms einer Zelle für die Analyse zugänglich gemacht wird, sind Proteine, die nicht in dem jeweiligen aufgereinigten Partialproteom enthalten sind, der weiteren Analyse nicht zugänglich. Zudem ist die Untersuchung einer topologischen Umverteilung von Proteinen nicht möglich. Das vorherige Aufteilen des Probenkollektivs und die parallele Herstellung verschiedener Teilproteome kann die oben genannten Nachteile nur bedingt beseitigen, da diese Variante zum einen sehr arbeitsaufwendig ist und zum anderen die Gefahr birgt, dass die Proben nicht homogen sind. Zudem ist die zur Verfügung stehende Probenmenge häufig so gering, dass eine Aufteilung der Probe unmöglich ist.

Wünschenswert wäre daher eine Methode, die es ermöglicht, die gesamte Probe in einem sequentiellen Verfahren in verschiedene Partialproteome aufzutrennen, wobei alle Proteine der Probe schlussendlich als Bestandteil eines der Partialproteome weiteren Analysen zugänglich sind.

Eine bekannte Methode zur sequentiellen Aufreinigung mehrerer Zellkompartimente ist die selektive Detergenz-Extraktion [1, 2], wobei vier Fraktionen (Partialproteome) erhalten werden. Im ersten Schritt wird eine cytosolische Fraktion erhalten, in der die löslichen Proteine des Cytosols und löslichen Komponenten des Cytoskeletts angereichert sind. Im zweiten Schritt erhält man eine Fraktion mit Proteinen der Membran/Organellen. Der dritte Schritt ergibt eine 'nukleäre' Fraktion, in der nach Angaben der Autoren Proteine der Kemmembran sowie lösliche nukleäre Proteine angereichert sein sollen. Im vierten und letzten Schritt entsteht eine Fraktion mit Proteinen des Detergenz-resistenten Cytoskeletts und der Kernmatrix.

Seit dem Publikationsdatum der ursprünglichen Methode im Jahre 1994 wurden verschiedene Modifikationen eingeführt, die sich alle ausschließlich auf den finalen Extraktionsschritt nach Solubilisierung des Zellkerns konzentrierten (zusammengefasst in Patton, 1999 [3]). Die beschriebene Methode wurde für die Untersuchung der subzellulären Redistribution eines Transkriptionsfaktors eingesetzt [4], welcher an der Signaltransduktion durch Cytokine beteiligt ist und nach der cytokin-induzierten Translokation in den Nukleus direkt an die DNA binden kann [5, 6, 7]. Nach den Ergebnissen der Autoren wurden jedoch insbesondere lösliche und/oder DNA-assoziierte Zellkern-Proteine gemeinsam mit Proteinen des Cytoskeletts und der nukleären Matrix erst im letzten und denaturierenden Extraktionsschritt erhalten.

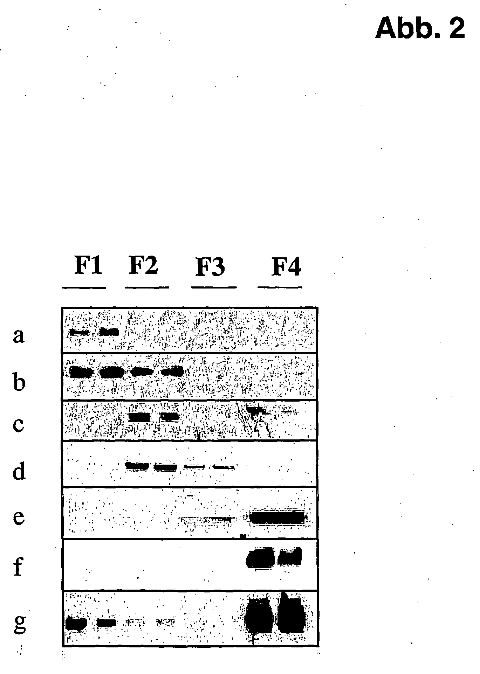

Sinibaldi RM et al., Journal of Biological Chemistry, Vol. 256, No. 21, 1981, Seiten 10735-10738, beschreiben die Präpäration von Proteinfraktionen des nuklearen Skeletts und anderen Zellkernfraktionen aus Drosophila Kc Zellen. Hierzu wird eine Nuklease-Fraktionierung_durchgeführt, die aus mehreren, sequentiell durchgeführten ZellkeRNextraktionen besteht, wobei insgesamt 4 verschiedene Zellkernfraktionen (3a,b und 4a,b in Abb. 2) erhalten werden, sowie ein Rückstand, der als "post-Nuklease Rückstand" bezeichnet wird.

Fey EG et al., Journal of Cell Biology, Bd. 98, Nr. 6, 1984, Seiten 1973-1984, offenbaren ein Verfahren zur Präparation nuklearer Matrix - intermediärer Filamente und einer Chromatin-Zellkernfraktion aus MDCK (Madin-Darby canine kidney) Zellen. Es wird eine 3-stufige Fraktionierung beschrieben, wobei zuerst ein Triton X-100 resistentes "Skelett-Netzwerk" isoliert wird, welches auch lösliche, Salz-labile Zellkernproteine enthält, und anschließend ein Salz-resistentes sowie ein Nuklease-resistentes Skelett-Netzwerk.

Dies bedeutet, dass nach den bisher bekannten Methoden für die sequentielle Aufreinigung mehrerer Zellkompartimente durch selektive Detergenz-Extraktion [1, 2, 3] keine eindeutige topologische Zuordnung nukleärer Proteine möglich ist, da keine eigenständige, von Komponenten des Cytoskeletts getrennte Fraktion erhalten wird, die insbesondere die Proteine des Zellkerninneren enthält.

Ein weiterer Nachteil der bekannten Extraktionsmethode ist, dass auch die Transkriptionsfaktoren in der vierten Fraktion enthalten sind. Da Transkriptionsfaktoren typischerweise in einer wesentlich geringeren Menge vorkommen als Cytoskelettproteine, wird ihre Detektion und Analyse durch die Anwesenheit der Cytoskelettproteine deutlich erschwert.

Zudem ist die nachfolgende funktionelle Analyse der in der vierten Fraktion enthaltenen Proteine stark eingeschränkt, weil bei der Gewinnung dieser Fraktion Natriumdodecylsulfat (SDS) eingesetzt wird. Dadurch werden die Proteine denaturiert und sind daher wichtigen Analyseverfahren, wie z.B. Co-Immunopräzipitation, Enzymaktivitätstests oder Electrophoretic Mobility Shift Assay (EMSA), nicht mehr zugänglich. Auch dies ist ein entscheidendes Hindernis für eine nachfolgende funktionelle Analyse dieser wichtigen regulatorischen Proteinklasse.

In keiner der seit 1994 vorgenommenen Anwendungen oder auch Veränderungen des Extraktionsprotokolls wurden diese Probleme erkannt, geschweige denn diesbezügliche Verbesserungen vorgeschlagen.

In eukaryontischen Zellen sind aber gerade die Proteine des Zellkerns von großer Bedeutung für verschiedenste Funktionen der Zelle. Der Zellkern macht etwa 10% des gesamten Volumens einer eukaryontischen Zelle aus und ist umgeben von einer nuklearen Hülle, welche aus zwei Membranen besteht. Die nukleare Hülle ist direkt mit dem endoplasmatischen Reticulum verbunden und ist mit zwei Netzwerken aus intermediären Filamenten verbunden: Erstens einer dünnen Schicht von Filamenten (nukleare Lamina) im Inneren des Zellkerns, welche die innere Zellmembran stützt, und Zweitens weniger regelmäßig organisierten intermediären Filamenten, welche die äußere nukleare Membran umgeben. Diese intermediären Filament-Netzwerke geben der Kernhülle somit eine gewisse Stabilität. Umgeben von der Kernhülle ist das Nukleoplasma, welches u.a. Chromatin (DNA und daran assozierte Proteine) und den Nukleolus enthält. Zu den Kernproteinen im Nukleoplasma, d.h. im Inneren des Zellkerns, gehören insbesondere DNA-bindende Proteine wie Histone sowie Nichthistonproteine (z.B. HMGs und Transkriptionsfaktoren), RNA-bindende Proteine, die beim RNA-Splicing und -Transport involviert sind, oder auch Skelett-assozierte Proteine. Die Proteine, die das Skelett im Inneren des Zellkerns, insbesondere die Lamina, bilden, werden bei dieser Betrachtung nicht als Teil des Nukleoplasmas bzw. des Zellkerninneren angesehen. Im folgenden werden daher als Proteine des Zellkerninneren insbesondere DNA-bindende Proteine wie Histone sowie Nichthistonproteine (z.B. HMGs und Transkriptionsfaktoren), RNA-bindende Proteine, die beim RNA-Splicing und -Transport involviert sind, oder auch Skelett-assozierte Proteine angesehen.

Verschiedene Methoden zur vereinzelten Solubilisierung von Zellkernproteinen sind in der Literatur beschrieben, u. a. durch Einsatz von hohen Salzkonzentrationen (>= 1M), wobei bekannt ist, dass hohe Salzkonzentrationen im Extraktionspuffer die Proteinwechselwirkungen und somit die weiteren funktionellen Analysen, wie z.B. die Analyse von Proteinkomplexen stören. Zusätzlich werden durch hohe Salzkonzentrationen in den extrahierten Fraktionen alle nachfolgenden proteinanalytischen Methoden wie **z.B.** Isoelektrische Fokussierung (IEF), Enzymaktivitätstests, EMSA, ELISA, SDS-PAGE und 2DE beeinträchtigt. Weiterhin werden durch hohe Salzkonzentrationen leicht Cytoskelett- und Cytoskelettassoziierte Proteine angegriffen, was zu einer Verunreinigung der Fraktion der Zellkernproteine mit Cytoskelett-Proteinen bewirken kann.

Aufgabe der vorliegenden Erfindung war es daher, ein Verfahren bereitzustellen, das im Zuge der sequentiellen Aufteilung des Gesamtproteoms in Partialproteome neben weiteren Partialproteomen auch die Erzeugung eines Partialproteoms der Proteine des Zellkerninneren ermöglicht, ohne die Proteine des Zellkerninneren dabei wesentlich zu denaturieren.

Es wurde gefunden, dass bei der Erstellung von Partialproteomen aus dem Gesamtproteom einer Zellpräparation, aus der nach Abtrennung der cytosolischen Proteine und der Proteine der Membran/Organellen unter anderem erhaltenen Zellkernpräparation, die Freisetzung der Proteine des Zellkerninneren effizient und überraschend einfach bewirkt wird, ohne dabei die Integrität des Cytoskeletts signifikant zu beeinträchtigen, wenn die Zellkernpräparation mit einer erfindungsgemäßen Salz- und Detergenzlösung sowie bevorzugt zusätzlich mit einer Nuklease extrahiert wird.

Da bei der Präparation des Partialproteoms der Proteine des Zellkerninneren die Proteine des Detergenz-resistenten Cytoskeletts und der nukleären Matrix nicht in wesentlichem Umfang mitextrahiert werden, können diese in einem nachfolgenden sequentiellen Verfahrensschritt als eigenständige Fraktion erhalten werden.

Gegenstand der vorliegenden Erfindung ist daher ein Verfahren zur sequentiellen Herstellung von Partialproteomen aus dem Gesamtproteom einer Zellpräparation, gekennzeichnet durch folgende Verfahrensschritte:
a) Bereitstellen einer Probe enthaltend eine Zellpräparation
b) Extraktion der cytosolischen Proteine und der Membran/Organellen-Proteine aus der in Schritt a) bereitgestellten Probe, wobei eine Zellkernpräparation zurückbleibt.
c) Extraktion der Proteine des Zellkeminneren aus der in Schritt b) erhaltenen Zellkernpräparation durch Behandlung mit einem Extraktionspuffer mit einem pH-Wert zwischen 6,5 und 8, der zumindest die folgenden Bestandteile enthält:
   insgesamt 0,1 bis 7 Massenprozent eines oder mehrerer nichtionischer Detergentien
   - insgesamt 0,05 bis 3 Massenprozent eines oder mehrerer Cholsäurederivate
   - eines oder mehrere Salze aus der Gruppe der Alkalimetall- und/oder Ammoniumsalze in einer Gesamtkonzentration zwischen 75 und 500 mMol/l,
   wobei Detergenz-resistente Proteine des Cytoskeletts und der Kernmatrix nicht in wesentlichem Umfang zusammen mit den Proteinen des Zellkerninneren extrahiert werden, sondern im Extraktionsrückstand verbleiben. Man erhält in Schritt c) als Extrakt demnach ein an Proteinen des Zellkerninneren angereichertes Partialproteom.

In einer bevorzugten Ausführungsform enthält der in Schritt c) eingesetzte Extraktionspuffer zusätzlich eine Nuklease.

In einer bevorzugten Ausführungsform enthält der in Schritt c) eingesetzte Extraktionspuffer Polyoxyethylensorbitanmonopalmitat (Tween^{®} 40) als nicht-ionisches Detergenz, Deoxycholat als Cholsäurederivat und NaCl als Alkalimetallsalz.

In einer bevorzugten Ausführungsform erfolgt die Extraktion der cytosolischen Proteine und der Membran/Organellen-Proteine in Schritt b) durch:
b i) Extraktion der cytosolischen Proteine aus der in Schritt a) bereitgestellten Probe durch selektive Permeabilisierung der Plasmamembran, ohne dabei die Integrität der subzellulären Membran/Organell Strukturen, des Zellkerns sowie des Cytoskeletts signifikant zu beeinträchtigen, durch Behandlung mit einem Saponin, Behandlung mit Streptolysin-O, enzymatischer Einwirkung mittels z.B. Lipase, Einwirkung mechanischer Kräfte wie z.B. Elektroporation, Einfrieren/Auftauen, Filter-"ripping", oder einer Kombination der genannten Möglichkeiten. Man erhält als Extrakt ein an cytosolischen Proteinen angereichertes Partialproteom.
b ii) Extraktion der Membran/Organellen-Proteine aus dem in Schritt b ii) nach der Extraktion verbliebenen Teil der Probe unter Erhaltung der strukturellen Integrität von Zellkern und Cytoskelett. Man erhält als Extrakt ein an Membran/Organellen-Proteinen angereichertens Partialproteom.

In einer bevorzugten Ausführungsform werden in einem zusätzlichen Verfahrensschritt d) aus dem in Schritt c) verbliebenen Extraktionsrückstand die Proteine des Detergenz-resistenten Cytoskeletts und der Kemmatrix als weiteres Partial-Proteom extrahiert.

Gegenstand der vorliegenden Erfindung ist zudem ein Kit für die Proteinextraktion zumindest enthaltend einen Extraktionspuffer mit einem pH-Wert zwischen 6,5 und 8, der zumindest die folgenden Bestandteile enthält:
- insgesamt 0,1 bis 7 Massenprozent eines oder mehrerer nicht ionischer Detergentien
- insgesamt 0,05 bis 3 Massenprozent eines oder mehrerer Cholsäurederivate
- eines oder mehrere Salze aus der Gruppe der Ammonium- und/oder Alkalimetallsalze in einer Gesamtkonzentration zwischen 75 und 500 mMol/l,
sowie einen Puffer zur Extraktion der cytosolischen Proteine, einen Puffer zur Extraktion der Membran/Organellen-Proteine und einen Puffer zur Extraktion der Proteine des Detergenz-resistenten Cytoskeletts und der Kemmatrix. Mit dieser bevorzugten Ausführungsform des Kits kann das erfindungsgemäße Verfahren zur Herstellung von vier Partialproteomen aus dem Gesamtproteom einer Zellpräparation durchgeführt werden.

In einer bevorzugten Ausführungsform enthält der Kit zusätzlich eine Nuklease, die dem Extraktionspuffer zugesetzt werden kann.

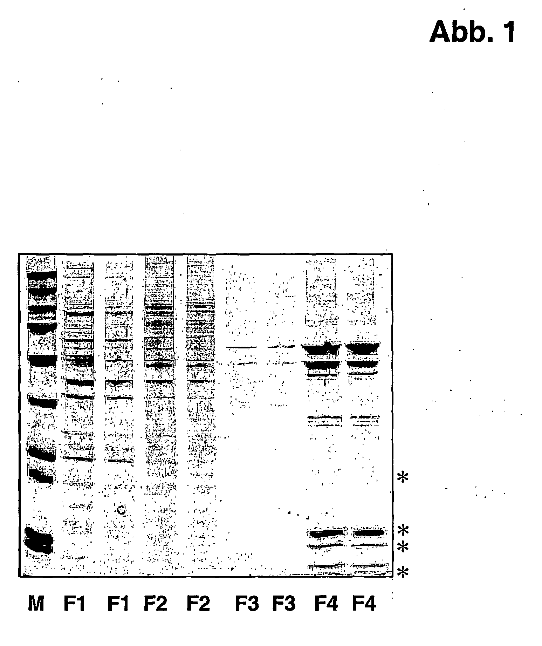

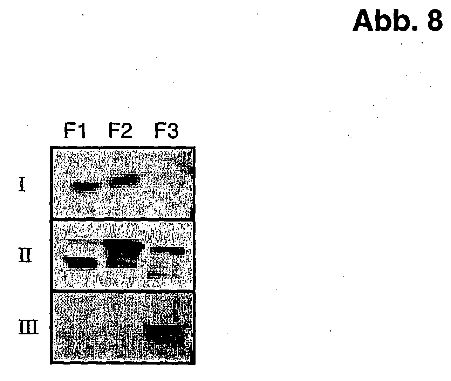

Die Erläuterungen zu den Abbildungen 1 bis 11 finden sich in den Beispielen 1 bis 12.

Das erfindungsgemäße Verfahren ermöglicht erstmals die Aufteilung des Gesamtproteoms einer Zellpräparation in mehrere subzelluläre Partialproteome, wobei eines der Partialproteome angereichert ist an Proteinen des Zellkerninneren. Somit wird eine topologische Zuordnung und funktionelle Analyse erstmalig durch ein Extraktionsverfahren ermöglicht.

Partialproteom der Proteine des Zellkerninneren bedeutet erfindungsgemäß ein Partialproteom, in dem hauptsächlich Proteine des Zellkerninneren angereichert sind. Das erfindungsgemäße Partialproteom der Proteine des Zellkerninneren enthält keinen oder nur einen die weiteren funktionellen Analysen nicht störenden geringen Anteil an Detergenz-resistenten Proteinen des Cytoskeletts und der nukleären Matrix. Weiterhin liegen die Proteine in dem nach dem erfindungsgemäßen Verfahren erhältlichen Partialproteom der Proteine des Zellkerninneren weitgehend nichtdenaturiert vor. Das bedeutet, durch den erfindungsgemäß in Schritt c) eingesetzten Extraktionspuffer wird eine Denaturierung der Proteine so weitgehend vermieden, dass ein ausreichender Anteil des in Schritt c) erhaltenen Partialproteoms der weiteren funktionellen Analyse zugeführt werden kann.

Als Zellpräparation können in dem erfindungsgemäßen Verfahren eukaryontische Zellen eingesetzt werden, beispielsweise in Suspension kultivierte Zellen oder adhärente Zellen. Auch Zellpräparate aus Gewebe können eingesetzt werde, wobei Zellen oder Zellgruppen erhalten werden, indem man Gewebe mittels biochemischem, enzymatischem und/oder physikalischem Mitteln zerlegt. Die Zellpräparation kann auch andere eukaryontische Zellen enthalten wie z.B. Spheroplasten aus Hefezellen, Pflanzenzellen nach Entfernung der Zellwand oder auch Insektenzellen sowie Zellpräparate von Pilzen. Bevorzugt werden in dem erfindungsgemäßen Verfahren als Zellpräparation eukaryontische Gewebekulturzellen von Säugern eingesetzt.

Als Zellkernpräparation werden erfindungsgemäß Rückstände der Zellpräparation bezeichnet, aus denen zuvor die cytosolischen Proteine sowie die Membran/Organellen-Proteine entfernt wurden. Bei der Entfernung dieser Proteine muss sichergestellt werden, dass die in der resultierenden Zellkernpräparation enthaltenen Zellkerne noch soweit intakt sind, dass die Proteine des Zellkerninneren noch überwiegend in der Zellkernpräparation vorliegen und nicht bereits bei der Entfernung der cytosolischen Proteine sowie die Membran/Organellen-Proteine entfernt wurden. Diese Bedingung wird beispielsweise durch die im folgenden für die Extraktion der cytosolischen Proteine sowie die Membran/Organellen-Proteine empfohlenen Puffersysteme gewährleistet. Weiterhin enthält eine Zellkernpräparation, die durch Entfernung der cytosolischen Proteine sowie der Membran/Organellen-Proteine aus Zellpräparationen hergestellt wurde, zumeist noch Bestandteile des Cytoskeletts.

Das erfindungsgemäße Verfahren ermöglicht die Aufteilung des Gesamtproteoms der Zellpräparation in mindestens drei Partialproteome, von denen eines angereichert ist mit den Proteinen des Zellkerninneren. Neben dem Partialproteom der Proteine des Zellkerninneren können unterschiedliche weitere Partialproteome erzeugt werden. Die Art der weiteren Partialproteome ist abhängig von der Art der in Schritt b) durchgeführten Extraktion sowie der im Anschluss an die Extraktion des Partialproteoms der Proteine des Zellkerninneren (Schritt c) vorgenommenen Extraktionsschritte.

Beispielsweise können die in Schritt b) extrahierten cytosolischen Proteine und Membran/Organellen-Proteine parallel in Form eines Partialproteoms extrahiert werden oder bevorzugt sequentiell, wobei zwei Partialproteome (ein Partialproteom angereichert mit cytosolischen Proteinen, ein Partialproteom angereichert mit Membran/Organellen-Proteinen) entstehen. "Angereichert" bedeutet erfindungsgemäß, dass in dem jeweiligen Partialproteom vorwiegend die als "angereichert" bezeichneten Proteine vorliegen. Andere Proteine sind nur in einer Menge vorhanden, die die weitere Analyse der angereicherten Proteine nicht wesentlich beeinträchtigt.

In einer bevorzugten Ausführungsform wird in Schritt b) zur Extraktion der cytosolischen Proteine die selektive Permeabilisierung der Plasmamembran der Zellen ohne signifikante Beeinträchtigung anderer zellulärer Substrukturen durch chemische Einwirkung mit einem Saponin, welches sich vorzugsweise in cholesterinreiche Membranen einlagert, insbesondere mit einem Digitonin-enthaltenden Puffer, bewirkt. Alternativ könnte dies auch mit anderen Substanzen wie z.B. Streptolysin-O, enzymatischer Einwirkung mittels z.B. Lipase oder auch Einwirkung mechanischer Kräfte mittels z.B. Elektroporation, Einfrieren/Auftauen, Filter-"ripping", oder deren Kombinationen bewirkt werden. Solche Verfahren sind in der Literatur beschrieben und der Fachmann bekannt. Man erhält als Extrakt ein an cytosolischen Proteinen angereichertes Partialproteom.

Nach der Extraktion der cytosolischen Proteine werden aus dem Extraktionsrückstand die Membran/Organellen-Proteine unter weitgehendem Erhalt der strukturellen Integrität von Zellkern und Cytoskelett extrahiert. Dafür eignen sich beispielsweise nicht-ionische- Detergentien oder zwitter-ionische Detergentien unter milden Bedingungen. Insbesondere Triton^{®} X-100 ist hierfür geeignet. Auch diese Verfahren sind in der Literatur beschrieben und dem Fachmann bekannt. Man erhält als Extrakt ein Partialproteom angereichert mit Membran/Orgariellen-Proteinen. Als Extraktionsrückstand erhält man eine Zellkernpräparation, die Verfahrensschritt c) zugeführt werden kann.

Sofern die Eigenschaften der Zellkernpräparation bezüglich der Erhaltung der strukturellen Integrität des Zellkerns bzw. der Zellkerne für Schritt c) nicht wesentlich verändert werden, kann in Schritt b) die Extraktion der cytosolischen und Membran/Organellen-Proteine auch mit anderen Mitteln durchgeführt werden. Weiterhin ist es möglich, Schritt b) auch in mehr als zwei Extraktionsschritten zur Erstellung von mehr als zwei Teilproteomen durchzuführen. Bevorzugt ist jedoch die Aufteilung in zwei Teilschritte zur Erstellung eines Partialproteoms, das mit cytosolischen Proteinen angereichert ist, und eines Partialproteoms, das mit Membran/Organellen-Proteinen angereichert ist.

Um in Schritt b) nur ein Partialproteom zu erzeugen, das sowohl die cytosolischen wie auch die Membran/Organellen-Proteine enthält, ist es ausreichend die Zellpräparation aus Schritt a) mit Reagenzien zu behandeln, wie sie oben zur Extraktion der Membran/Organellen-Proteine beschrieben wurden, d.h. mit nicht-ionischen Detergentien oder zwitter-ionischen Detergentien unter milden Bedingungen. Hierbei werden Detergentien mit einem HLB-Wert zwischen 12 und 20 für die nicht denaturierende Solubilisierung von Membranproteinen bevorzugt.
Insbesondere Detergentien mit hoher Aggregatzahl, wie z. B. Triton^{®} X-100 und NP-40, sind hierfür geeignet. Eine vorherige Behandlung zur selektiven Extraktion von cytosolischen Proteinen (wie Behandlung mit einem Digitonin- enthaltenden Puffer oder mit Streptolysin-O, enzymatische Einwirkung mittels z.B. Lipase oder auch Einwirkung mechanischer Kräfte mittels z.B. Elektroporation, Einfrieren/Auftauen, Filter-"ripping", oder deren Kombinationen) sind im allgemeinen nicht notwendig.

Die nach der Extraktion der cytosolischen Proteine und der Membran/Organellen-Proteine erhaltene Zellkernpräparation wird dann erfindungsgemäß in Schritt c) der selektiven Detergenzextraktion der Proteine des Zellkerninneren zugeführt.

Für das erfindungsgemäße Verfahren ist bei Schritt c), der Herstellung eines Partialproteoms, das mit den Proteinen des Zellkerninneren angereichert ist, von großer Bedeutung, dass die Proteine des Zellkerninneren größtenteils an Bindungspartner, wie Nukleinsäuren oder andere Proteine, gebunden vorliegen. Die Stärke der Bindung, welche die Proteine des Zellkerninneren mit ihren jeweiligen Bindungspartnern eingehen, ist sehr unterschiedlich und teilweise abhängig von dem vorliegenden Milieu. So ist z.B. die Interaktion von Histonen mit DNA-Molekülen deutlich stärker als die von HMGs. In dem erfindungsgemäßen Verfahren kann die Bindung der Proteine des Zellkerninneren an ihre Bindungspartner, insbesondere Nukleinsäuren, gezielt für eine weitere Selektion der Proteine des Zellkerninneren eingesetzt werden. Je nach Wahl des Extraktionspuffers können vermehrt leicht gebundene, stark gebundene Proteine des Zellkerninneren oder alle Proteine des Zellkerninneren nahezu unabhängig von ihrer Bindungsstärke an die jeweiligen Bindungspartner, insbesondere Nukleinsäuren, extrahiert werden.

Der in Schritt c) verwendete Extraktionspuffer weist typischerweise einen pH-Wert zwischen 6,5 und 8 auf. Bei niedrigeren pH-Werten können insbesondere Probleme mit der Löslichkeit bestimmter Bestandteile, wie der nicht-ionischen Detergenzien oder der Cholsäurederivate, auftreten. Der bevorzugte pH-Bereich liegt zwischen pH 6,9 und pH 7,8. Als Puffersubstanzen eignen sich daher solche, die im schwach sauren bis schwach alkalischen Bereich puffern, wie MOPSO, BES, MOPS, Phosphat oder bevorzugt PIPES. Die Pufferkonzentration beträgt typischerweise zwischen 2-100 mM, bevorzugt zwischen 5 und 20 mM.

Weiterhin enthält der erfindungsgemäß verwendete Extraktionspuffer ein oder mehrere geeignete nicht-ionische Detergentien in einem Anteil von typischerweise insgesamt 0,1 bis 7 Massen%, auf jedem Fall oberhalb der CMC, bevorzugt zwischen 0,2 und 5 Massen%. Eine Erhöhung der Detergenzkonzentration über den angegebenen Bereich hinaus birgt die Gefahr, dass eine vermehrte Denaturierung von Proteinen eintritt. Zudem können durch hohe Detergenzkonzentrationen die späteren Analyseverfahren gestört werden.
Wichtige Merkmale zur Unterscheidung von Detergentien sind beispielsweise der HLB-Wert, CMC und die Aggregatzahl.

Erfindungsgemäß geeignete nicht-ionische Detergentien sind solche, die in der erfindungsgemäß gewählten Konzentration in Kombination mit den anderen Bestandteilen des Extraktionspuffers bewirken, dass die bislang vorhandene Struktur des Zellkerns aufgelöst wird. Wichtig ist dabei, dass die vorher vorhandene Morphologie des Zellkerns zerstört wird und die Nukleoplasma-Proteine freigesetzt werden, ohne dass ein wesentlicher Anteil der Proteine des Cytoskeletts freigesetzt wird. Erfindungsgemäß besonders gut geeignete nicht-ionische Detergentien sind z. B. solche mit einer hydrophilen Polyoxyethylen Kopf-Gruppe, welche keinen Phenylring zwischen Alkylkette und Kopfgruppe besitzen, bevorzugt Tween^{®}-Detergentien, insbesondere Polyoxyethylensorbitanmonopalmitat (Tween^{®} 40).

Ein weiterer wesentlicher Bestandteil des erfindungsgemäß verwendeten Extraktionspuffers sind ein oder mehrere Cholsäurederivate. Erfindungsgemäß geeignete Cholsäurederivate sind anionische Detergentien. Sie besitzen ein Steroidgrundgerüst, das eine oder mehrere gleiche oder verschiedene Seitenketten, wie z.B. -OH, -CH₃, C₂H₅ oder auch beispielsweise Aminosäuren, trägt, sowie eine Carboxylgruppe am Ende einer Alkylkette. Besonders geeignet sind Cholsäurederivate, die - im Gegensatz zu nicht-ionischen Detergentien wie Triton - eine niedrige Aggregatzahl besitzen. Als Cholsäurederivate gelten erfindungsgemäß auch Cholsäure selbst sowie Salze der Cholsäure. Bevorzugt wird als Cholsäurederivat Na-Deoxycholat eingesetzt. '

In dem erfindungsgemäß in Schritt c) eingesetzten Extraktionspuffer werden ein oder mehrere Cholsäurederivate in einem Anteil von typischerweise insgesamt 0,05 bis 3 Massenprozent, bevorzugt 0,1 bis 2,5 Massen% verwendet.Auch hier kann eine Erhöhung des Anteils über den angegebenen Bereich eine Denaturierung der Proteine und/oder eine Störung der weiteren Analysen bewirken.

Weiterhin enthält der Extraktionspuffer ein oder mehrere Alkalimetall-und/oder Ammoniumsalze in einer Konzentration zwischen 75 und 500 mMol/I. Eine Erhöhung der Salzkonzentration über den angegebenen Konzentrationsbereich kann eine Denaturierung der Proteine und/oder eine Störung der weiteren Analysen bewirken.

Erfindungsgemäß bevorzugt werden Alkalimetallsalze, insbesondere Natrium-Salze, wie Nitrate, Sulfate, Phosphate und besonders Halogenide, wie Bromide oder Chloride. Besonders bevorzugt ist NaCl.

Ein erfindungsgemäß besonders bevorzugter Extraktionspuffer für Schritt c) enthält ca. 10 mM PIPES, ca. 1 Massen% Tween^{®} 40, ca. 0,5 Massen% Na-Deoxycholat und ca. 350 mM NaCl. Zusätzlich kann der Extraktionspuffer für Schritt c) weitere Bestandtteile, wie z.B. Stabilisatoren oder Konservierungsmittel enthalten.

In einer bevorzugten Ausführungsform des Verfahrens enthält der Extraktionspuffer für Schritt c) zusätzlich eine Nuklease, bevorzugt eine Endonuklease aus *Serratia marcescens* (Benzonase^{®} der Firma Merck KGaA, Darmstadt). Für die Aktivität der Nukleasen ist es zumeist vorteilhaft, dem Extraktionspuffer zusätzlich Erdalkalimetallsalze, typischerweise in einer Konzentration zwischen 0,02 und 10 mMol/l, zuzusetzen. Besonders geeignete Salze und deren Konzentration sind je nach der verwendeten Nuklease zu wählen. Beim Einsatz von Benzonase® ist beispielsweise MgCl₂ in einer Konzentration zwischen 1 und 10 mM, insbesondere zwischen 1 und 2 mM geeignet.

Die geeignete Menge bzw. Aktivität der eingesetzten Nuklease richtet sich nach der angestrebten Dauer des Extraktionsschrittes. Je mehr Nuklease zugesetzt wird, desto schneller kann die Extraktion durchgeführt werden. Dabei ist jedoch zu bedenken, dass das bei der Extraktion erhaltene Partialproteom auch mit der Nuklease verunreinigt ist. Je mehr Nuklease eingesetzt wird, desto mehr Nuklease ist auch in dem Partialproteoms, das mit den Proteinen des Zellkerninneren angereichert ist, enthalten. Es ist zu beachten, dass Nukleasen bestimmte Verfahren der Proteinanalyse, wie z.B. EMSA, stören. Soll das Partialproteom, das mit den Proteinen des Zellkerninneren angereichert ist, einem solchen Analyseverfahren zugeführt werden, muss auf den Zusatz von Nukleasen verzichtet werden.

Wie aus Beispiel 4 und 5 ersichtlich wird, kann durch den Zusatz von Nuklease beeinflusst werden, ob eher schwach gebundene oder stark gebundene Proteine des Zellkerninneren bevorzugt extrahiert werden. Wird dem Puffer keine Nuklease zugesetzt, so reduziert sich der Anteil der stark Nukleinsäure-gebundenen Proteine, wie der Histone.

Die Integrität des Cytoskeletts wird durch den erfindungsgemäßen Verfahrensschritt c) zur Erstellung eines Partialproteoms der Proteine des Zellkerninneren nicht signifikant beeinträchtigt, so dass durch das erfindungsgemäße Verfahren erstmals eine von den hoch-abundanten Komponenten des Cytoskeletts getrennte, in den niedrig-abundanten nukleären Proteinen angereicherte Fraktion erhalten werden kann.

Zusätzlich können im Anschluss an Schritt c) in einem nachfolgenden Extraktionsschritt aus dem verbleibenden Extraktionsrückstand nach bekannten Methoden die Proteine des Detergenz-resistenten Cytoskeletts und der Kernmatrix als weiteres Partialproteom extrahiert werden. Geeignet sind dafür z.B. SDS-haltige denaturierende Puffer. Diese Puffer können zusätzlich reduzierende Agenzien wie z. B. DTT oder β-Mercaptoethanol enthalten.

Das erfindungsgemäße Verfahren ist einfach, effizient und automatisierbar und vereinfacht die Detektion und Analyse der niedrig-abundanten und gleichzeitig pharmakologisch interessantesten regulatorischen Proteinklassen (Membranproteine wie z.B. plasmamembranständige Rezeptoren oder DNA-assoziierte Proteine und Transkriptionsfaktoren) wesentlich. Dies wird durch einfache und selektive Abtrennung von hoch-abundanten Proteinen (z.B. Haushaltsproteine im Cytosol als auch Cytoskelettproteine) erreicht. Das Verfahren erlaubt erstmals die Betrachtung funktioneller Proteine von Zellkern getrennt vom Cytoskelett ohne den Verlust anderer Partialproteome. Die erhaltene Partialproteome können einer Vielzahl von etablierten Proteinanalyse-Techniken zugeführt werden. Dies erlaubt z.B. auch Funktionsuntersuchungen mittels Enzym Aktivitätsessays, oder *Electrophoretic Mobility Shift Assay* (EMSA) für Zellkernproteine. Außerdem ermöglicht das erfindungsgemäße Verfahren nicht nur die topologische Zuordnung zellulärer Proteinen, sondern auch die Analyse der dynamischen Umverteilung der genannten Proteinklassen in den verschiedenen zellulären Kompartimenten inklusive des Zellkerns, was durch bisherige Ansätze nicht möglich war. Eine solche Analyse ergibt wichtige Information über die Funktion des untersuchten Proteins. Verknüpft mit gängigen Techniken wie z.B. der Massenspektrometrie (MS), 2D-Gelelektrophorese (2DE), Aminosäure-Sequenzierung oder Immunoblotting kann die umfassende Analyse der in der Zelle vorhandenen Proteine und deren subzellulärer Verteilung eingesetzt werden, um neue Proteine oder Proteinfunktionen zu identifizieren und auf deren Beteiligung in bestimmten zellulären Signalwegen hinzuweisen. Das erfindungsgemäße Verfahren stellt somit einen grundlegenden Ansatz dar, um sowohl physiologische als auch pathophysiologische zelluläre Vorgänge oder Veränderungen zu charakterisieren, welche sich u.a. durch Krankheiten oder unter Einfluss von Medikamenten/Substanzen ergeben.

Gegenstand der vorliegenden Erfindung ist zudem ein Kit für die Proteinextraktion, insbesondere für die Erstellung von Partialproteomen aus einem in einer Zellpräparation enthaltenen Gesamtproteom, zumindest enthaltend einen Extraktionspuffer mit einem pH-Wert zwischen 6,5 und 8, der zumindest die folgenden Bestandteile enthält:
- insgesamt 0,1 bis 7 Massenprozent eines oder mehrerer nicht ionischer Detergentien
- insgesamt 0,05 bis 3 Massenprozent eines oder mehrerer Cholsäurederivate
- eines oder mehrere Salze aus der Gruppe der Ammonium-und/oder Alkalimetallsalze in einer Gesamtkonzentration zwischen 75 und 500 mMol/l.

Die für den Extraktionspuffer für Schritt c) angegebenen bevorzugten Mengen der einzelnen Bestandteile gelten ebenso für den Extraktionspuffer des Kits. Der Puffer liegt typischerweise als trockene Reagenzienmischung zum Auflösen in Wasser, als wässriges Konzentrat oder zum direkten Einsatz in wässriger Form vor.

In einer bevorzugten Ausführungsform enthält der Kit weiterhin eine Nuklease zum Zusatz zu dem Extraktionspuffer.

In einer bevorzugten Ausführungsform enthält der Kit zusätzlich Puffer zur Extraktion der cytosolischen Proteine und/oder der Membran/Organellen-Proteine aus Zellpräparaten. Besonders bevorzugt enthält der Kit Reagenzien zur Durchführung der bevorzugten Ausführungsform des erfindungsgemäßen Verfahrens, bei der aus dem Gesamtproteom einer Zellpräparation vier Partialproteome (Partialproteom angereichert mit cytosolischen Proteinen, Partialproteom angereichert mit Membran/Organellen-Proteinen, Partialproteom angereichert mit Proteinen des Zellkerninneren, Partialproteom angereichert mit Proteinen des Cytoskeletts und der Kernmatrix) erhalten werden.

Genauso kann der Kit weitere Bestandteile bzw. Reagenzien aufweisen, wie zur Durchführung des erfindungsgemäßen Verfahrens geeignete Geräte und Verbrauchsgegenstände und/oder Reagenzien zur weiteren Bearbeitung der Partialproteome, z.B. für die funktionelle Analyse oder Lagerung.

### Literaturverzeichnis

[1] Ramsby et al (1994): "Differential detergent fractionation of isolated hepatocytes" Electrophoresis 15(2) 265-77
[2] Ramsby et al (1999): "Differential detergent fractionation of eukaryotic cells" in Methods in Molecular Biology, Volume 112: "2-D proteome Analysis Protocols" Andrew J. Link (Editor, Humana Press Totowa New Jersey, 1999
[3] Patton W.F. (1999): "Protein subcellular redistribution: linking physiology to genomics via the proteome and separation technologies" J. Chrom B, 722, 203-223
[4] Chiang et al. (2000): "NFkappaB translocation in human microvessel endothelial cells unsing a four-compartment subcellular protein redistribution assay" J.Biochem.Biophys Meth. 46, 53-68
[5] Jimi et al. (1998): "Activation of NFkappaB is involved in the survival of osteoclasts promoted by interleuin-1" J. Biol. Chem 273, 8799-805
[6] Mejdoubi et al. (1999): "NFkappaB is involved in the induction of the rat hepatic alpha-1 acid glycoprotein gene by phenobarbital" Biochem Biophys Res. Commun. 254, 93-9
[7] Butcher et al (2001): "Toxoplasma gondii tachyzoites inhibit proinflammatory cytokine induction in infected macrophages by preventing nuclear translocation of the transcription factor NFkappaB" J. Immun. 167(4), 2193-201
[8] Ott et al (2001): "Accuracy of two-dimensional electrophoresis for target discovery in human colorectal cancer" The pharmacogenomics Journal-Nature 1 (2), 142-51
[9] Lehmann et al (2001): " Tomato ribonuclease LX with the functional endoplasmic reticulum retention motif HDEF is expressed during programmed cell death processes, including xylem differentiation, germination, and senescence". Plant-Physiol. 127 (2), 436-49
[10] Egesten et al (1997): " Ribonucleases and host defense: identification, localization and gene expression in adherent monocytes in vitro". Biochim-Biophys-Acta. 1358 (3), 255-60
[11] Frank et al (1998): "Cloning, subcellular localization and functional expression of human RNase HII". Biol-Chem. 379 (12), 1407-12
[12] Dignam et al (1983):"Accurate transcription initiation by RNA polymerase II in a soluble extract from isolated mammalian nuclei" Nucleic-Acids-Research 11 (5), 1475-89

### Abkürzungsverzeichnis

| | |
|---|---|
| *µ* | Mikro |
| 2-DE | Zweidimensionale Gelelektrophorese |
| A431 | Epithelkarzinomzellen |
| Bes | N,N-Bis(2-hydroxyethyl)-2-aminoethansulfonsäure |
| CHO | chinese hamster ovary |
| CMC | Kritische Micellare Konzentration |
| DAPI | 4',6-Diamidino-2-phenylindole |
| DNA | Desoxyribonucleinsäure |
| DOC | Deoxycholat |
| DTT | Dithiothreitol |
| EDTA | Ethylendiamintetraessigsäure |
| ELISA | enzyme linked immunosorbent assay |
| EMSA | Electrophoretic Mobility Shift Assay |
| F1 | Fraktion 1, angereicherte cytosolische Proteine |
| F2 | Fraktion 2, angereicherte Membran/Organellen Proteine |
| F3 | Fraktion 3, angereicherte Kernproteine |
| F4 | Fraktion 4, angereicherte Cytoskelettproteine |
| H1, H2,...,H4 | Histon1, Histon2, ..., Histon4 |
| HEPES | [4-(2-Hydroxyethyl)-piperazino]-ethansulfonsäure |
| HLB-Wert | Hydrophile/Hydrophobe-Balance |
| HMG | High Motility Group |
| HSP70 | Hitze-Schock-Proteine 70 kDa |
| IEF | Isoelektrische Fokussierung |
| KDa, kD | Kilodalton |
| L | Liter |
| M | Molar |
| mA | Milliampere |
| MCF7 | Mammakarzinomzellen |
| Mes | 2-Morpholinoethansulfonsäure |
| mg | Milligramm |
| MgCl₂ | Magnesiumchlorid |
| min | Minuten |
| Mio | Millionen |
| mMol/I | millimol pro Liter |
| MOPS | 3-Morpholino-1-propansülfonsäure |
| MOPSO | 3-(N-Mopholino)-2- ' hydroxypropansulfonsäure |
| MS | Massenspektrömetrie |
| mV | Milli Volt |
| MW | Molecular Weight |
| NaCl | Natriumchlorid |
| Na-DOC | Natrium-deoxycholat |
| NP-40 | Polyethylenglycol-p-isooctylphenylether |
| pl | Isoelektrische Punkt |
| PIPES | Piperazine-1,4-bis(2-ethansulfonic acid) |
| PVDF | Polyvinylidendifluorid |
| RNA | Ribonukleinsäure (ribonucleic acid) |
| RT | Raumtemperatur |
| SAOS 2 | Osteosarkomzellen |
| SDS | Sodiumdodecylsulfat |
| SDS-PAGE | Sodiumdodecylsulfat-Polyacrylamidgelelektrophorese |
| T₂₅-Kulturflasche | Kulturflasche mit 25 cm² Fläche |
| T₇₅-Kulturflasche | Kulturflasche mit 75 cm² Fläche |
| Tris | 2-Amino-2-(hydroxymethyl)-1,3-propandiol |
| Triton^{®} X-100 | Octylphenoxypolyethoxyethanol |
| Tween^{®} 40 | Polyoxyethylensorbitanmonopalmitat |
| U | Unit |
| ÜN | über Nacht |
| V | Volt |
| v/v | Volume/Volume |
| w/v | weight/Volume |
| w/v | weight/volume |
| WB | Westem-Blotting |

### Beispiele

### Beispiel 1: Untersuchung des Proteinmusters mittels SDS-PAGE von Extrakten nach selektiver Detergenz-Extraktion von eukaryontischen Zellen nach dem Stand der Technik [1, 2, 3].

Das Ausgangsmaterial für diesem Versuch waren Mammakarzinomzellen (MCF7), die zu 80% konfluent als adhärenter Zellrasen auf einer Gewebekulturschale vorlagen. Die Durchführung der Extraktionsschritte fand nach dem Stand der Technik statt. Auf diese Weise entstanden die Proteinextrakte F1-F4. Diese Proteinextrakte wurden volumenäquivalent durch ein 12%-iges Polyacrylamidgel im elekrischen Feld aufgetrennt und mit Coomassie-Brilliant-Blue visualisiert. Ein solches Beispiel zeigt die Abbildung 1.
In Abbildung 1 werden folgende Bezeichnungen verwendet:
M=Marker. F1 bezeichnet die Fraktion 1 mit den cytosolischen Proteinen. F2 steht für Fraktion 2 und stellt die Membran/Organellen-Proteine dar. F3 demonstriert die im Stand der Technik [1, 2, 3] als 'nukleäre' Fraktion bezeichnete Fraktion 3 und F4 kennzeichnet Fraktion 4, die nach dem Stand der Technik die Proteine des Cytoskeletts und der Kernmatrix enthalten soll.

Auffällig an den bei der Reproduktion des Standes der Technik erhaltenen Ergebnissen (s. Abbildung 1) ist der im Vergleich zu den Fraktionen 1, 2 und 4 sehr geringe Proteingehalt der 'nukleären' Fraktion 3. Weiterhin finden sich die typischen Bandenmuster der DNA-assoziierten Histonproteine (die zur Gruppe der Proteine des Zellkerninneren gehören), in Fraktion 4, zusammen mit den Proteinen des Cytoskeletts. Die typischen Bandenmuster der DNA-assoziierten Histonproteine sind in Abbildung 1 mit einem * gekennzeichnet.
Dieses Ergebnis unterstützt die Feststellung der Tatsache, dass die in der Literatur [1, 2, 3] beschriebene Methode zur Herstellung von vier Partialproteomen kein Partialproteom liefert, in dem die Proteine des Zellkerninneren deutlich getrennt von den Proteinen des Cytoskeletts angereichert sind. Die nach dem Stand der Technik erhältliche Fraktion 3 ist nicht angereichert mit den Proteinen des Zellkerninneren. Diese finden sich vielmehr größtenteils in Fraktion 4, in der zum einen die Proteine nur denaturiert erhalten werden und zum anderen die Proteine des Cytoskeletts extrahiert werden.

### Beispiel 2: Immunoblotting-Untersuchung der topologischen Zuordnung von Markerproteinen in Extrakten nach selektiver Detergenz-Extraktion von eukaryontischen Zellen nach dem Stand der Technik [1, 2, 3]

Die entsprechend Beispiel 1 gewonnenen Proteinextrakte wurden mittels SDS-PAGE aufgetrennt und anschließend auf einer PVDF-Membran geblottet und mit ausgesuchten Markerproteine untersucht.

Die Markerproteine des Cytosols (a-b) und der Membran/Organellen (b-d) werden nach der Extraktion eines Zellpräparates nach dem Stand der Technik in den entsprechenden Fraktionen (F1 bis F4) detektiert (vergl. Abb. 2). Markerproteine des Zellkerninneren jedoch werden nach der Extraktion eines Zellpräparates nach dem Stand der Technik nicht in der entsprechenden, als 'nukleäre' Fraktion bezeichneten, Fraktion F3 erhalten. Der Transkriptionsfaktor c-jun (e) sowie das DNA-assoziierte Protein Histon 1 (f) (beides Proteine des Zellkerninneren) werden nicht in der'nukleären' Fraktion F3 detektiert, sondern werden erst in der letzten Fraktion (F4) gemeinsam mit dem hoch-abundanten Cytoskelettmarker Cytokeratin (g) unter denaturierenden Bedingungen freigesetzt. a: Calpain; b: HSP 70; c: Cadherin; d: Cytochrome P 450 reductase; e: c-jun; f: Histone; g: Cytokeratin

Die lmmunoblotanalyse der erhaltenen Fraktionen für repräsentative Markerproteine nach Detergenz-Extraktion nach dem Stand der Technik demonstriert, dass keine eigenständige Fraktion für die löslichen und DNA-assoziierten Proteine des Zellkerninneren erhalten wird. Vielmehr werden diese unter denaturierenden Bedingungen zusammen mit Proteinen des Cytoskeletts solubilisiert. Somit ist mit der Methode nach dem Stand der Technik z.B. eine eindeutige Analyse der topologischen Umverteilung der Proteine des Zellkerninneren nicht möglich.

### Beispiel 3: Beispielhafte Darstellung der selektiven Detergenz-Extraktion nach dem erfindungsgemäßen Verfahren

Die Extraktionspuffer wurden laut Tabelle unter 3a angesetzt. Die Durchführung der Extraktion ist unter 3b ausführlich beschrieben. Die Ergebnisse des erfindungsgemäßen Verfahrens werden in den folgenden Beispielen näher beschrieben.

### a. Pufferzusammensetzung, welche für das 3.b dokumentierte Experiment verwendet wurde

| **Puffer** | **Chemikallen** | **Konzentration** |
|---|---|---|
| EXTRAKTIONSPUFFER I | PIPES | 10 mM |
| pH 6,8 | Digitonin | 0,02% |
| | Saccharose | 300 mM |
| | Natriumchlorid | 15 mM |
| | EDTA | 0,5 mM |
| EXTRAKTIONSPUFFER II | PIPES | 10 mM |
| pH 7,4 | Triton X-100 | 0,50% |
| | Saccharose | 300 mM |
| | Natriumchlorid | 15 mM |
| | EDTA | 0,5 mM |
| EXTRAKTIONSPUFFER III | PIPES | 10 mM |
| pH 7,4 | Tween-40 | 1,0% |
| | Deoxycholat | 0,5% |
| | Natriumchlorid | 350 mM |
| | Magnesiumchlorid | 1 mM |
| | Benzonase | 500 U/ml |
| EXTRAKTIONSPUFFER IV | Sodiumdodecylsulfat (SDS) | 5% |
| pH 7,4 | Na2HPO4 | 10 mM |
| | NaH2PO4 | 10 mM |

### b. Beispielhafte Durchführung der Extraktion adhärenter Zellen

Die subzelluläre Fraktionierung wurde mit Gewebekulturzellen in T₂₅-Gewebekulturschalen durchgeführt. Bei der Verwendung anderer Kulturgefäße können die Puffermengen entsprechend maßstabsgerecht an die Fläche der verwendeten Platte angepasst werden. Für die Extraktion müssen vitale Zellen in der logarhytmischen Wachstumsphase bei etwa 80 % Konfluenz eingesetzt werden. In diesem Beispiel sind SAOS 2-Zellen verwendet worden.
1. Die Extraktionspuffer I, II und III auf Eis, die Extraktionspuffer IV und den Protease Inhibitor Cocktail bei RT auftauen.
2. Die Gewebekulturschale leicht ankippen, so dass das Medium aus der Kulturflasche entnommen werden kann, ohne den Zellrasen zu berühren. Medienreste sorgfältig entfernen.
3. Die Gewebekulturschale leicht ankippen. Vom Rand her 2 ml eiskalten Waschpuffer vorsichtig zupipettieren und den Zellrasen damit überschichten. Die Kulturschale für 5 min unter sanftem Schütteln bei 4°C inkubieren.
4. Die Gewebekulturschale leicht ankippen und den Waschpuffer sorgfältig entfernen, ohne den Zellrasen zu berühren.
5. Schritte 3 und 4 wiederholen, um Mediumbestandteile wie z. B. BSA, Aminosäuren und Indikatorfarbstoffe vollständig zu entfernen.
6. 1 ml eiskalten Extraktionspuffer I und 5 *µ*l Protease Inhibitor Cocktail miteinander vermischen. Vorsichtig vom Rand her auf die gewaschene Gewebekulturschale pipettieren, ohne den Zellrasen zu berühren. Die Zellen vorsichtig mit dem Puffer überschichten. Unter sanftem Schütteln für 10 min bei + 4° C inkubieren.
7. Die Gewebekulturschale vorsichtig ankippen. Die cytosolischen Proteinextrakte (Fraktion 1) vorsichtig aus der Kulturflasche entnehmen, ohne den Zellrasen zu berühren, und bis zur Verwendung am selben Tag auf Eis aufbewahren. Zur langfristigen Lagerung in geeigneten Mengen (z. B. 100 µl) aliquotieren und bei -80° C aufbewahren.
8. 1 ml eiskalten Extraktionspuffer II und 5 µl Protease Inhibitor Cocktail miteinander vermischen. Vorsichtig vom Rand her auf die Gewebekulturschale pipettieren, ohne den Zellrasen zu berühren. Die Zellen vorsichtig mit dem Puffer überschichten danach 30 min bei leichtem Schütteln und +4° C inkubieren
9. Die Gewebekulturschale vorsichtig kippen. Den Extrakt mit den Membran/Organellen-Proteinen (Fraktion 2) vorsichtig aus der Kulturflasche entnehmen, ohne den Zellrasen zu berühren, und bis zur Verwendung am selben Tag auf Eis inkubieren. Zur langfristigeren Lagerung in geeigneten Mengen aliquotieren und bei -80° C aufbewahren.
10. 500 *µ*l eiskalten Extraktionspuffer III mit 5 *µ*l Protease Inhibitor Cocktail und 10 *µ*l (250 U) Benzonase® miteinander vermischen und vorsichtig auf die restlichen Zellbestandteile pipettieren. Unter sanftem Schütteln für 10 min bei 4°C inkubieren.
11. Die Gewebekulturflache vorsichtig kippen. Den Proteinextrakt mit den nukleären Proteinen (Fraktion 3) vorsichtig aus der Kulturflasche entnehmen, ohne den Zellrasen zu berühren, und bis zur Verwendung am selben Tag auf Eis aufbewahren. Zur langfristigen Lagerung in geeigneten Mengen aliquotieren und bei -80° C aufbewahren.
12. 500 µl auf Raumtemperatur temperierten Extraktionspuffer IV und 5 µl Protease Inhibitor Cocktail miteinander vermischen und auf den restlichen Zellrasen gegeben. Die Zellschicht löst sich bei der Zugabe dieses Puffers von der Platte ab.
13. Durch Hinauf- und Hinunterpipettieren mit einer 1 ml-Pipettenspitze (z. B. Eppendorf) suspendieren. Nach vollständigem Lösen der restlichen Zellbestandteile in ein Mikrozentrifugenröhrchen transferieren und bis zur Verwendung am selben Tag auf Eis aufbewahren. Fraktion 4 enthält Cytoskelettproteine. Zur langfristigen Lagerung in geeigneten Mengen aliquotieren und bei -80° C aufbewahren.

### Beispiel 4: Morphologische Darstellung der Zellen bei der erfindungsgemäßen Durchführung der selektiven Detergenz-Extraktion

Analog zu Beispiel 3 wurde die Proteinextraktion aus einer Zellkultur in einer T₇₅-Kulturflasche (80% mit SAOS 2-Zellen konfluent bewachsen) durchgeführt. Diese wurden vor der Extraktion, d.h. unbehandelt (Bild i), und schrittweise nach der jeweiligen selektiven Detergenz-Extraktion (Bild ii-iv) auf verfahrensbedingte morphologische Veränderung mikroskopisch untersucht.

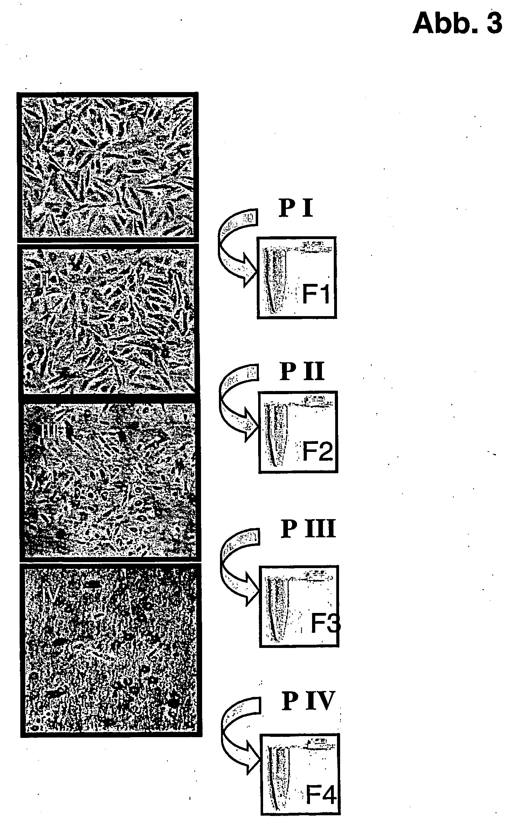

Abbildung 3 zeigt ein solches Beispiel. Nach der Behandlung der Zellen mit Extraktionspuffer I (Bild ii), treten die cytosolischen Inhalte der Zellen aus, wobei die Zellen schrumpfen. Die Integrität der Zellkerne und Plasmamembran bleibt weitestgehend erhalten. Nach der Inkubation der Zellen mit dem Extraktionspuffer II,(Bild iii) werden die Membran/Organellen-Proteine extrahiert, ohne die Integrität der Zellkerne zu beeinträchtigen. Nach der Inkubation des restlichen Zellmaterials mit dem Extraktionspuffer III (erfindungsgemäßer Verfahrensschritt c)) (Bild iv) wird die Fraktion der Proteine des Zellkerninneren extrahiert. Das restliche Zellmaterial wird im Extraktionspuffer IV aufgenommen. Man erhält eine Fraktion, in der die Cytoskelettbestandteile enthalten sind.
PI: Extraktionspuffer I
P II: Extraktionspuffer II
P lll: Extraktionspuffer III
P IV: Extraktionspuffer IV

i: SAOS2- Zellen unbehandelt
ii: SAOS2-Zellen mit Extraktionspuffer I behandelt, nach Inkubation erhält man Fraktion 1 (F1)
iii: SAOS2-Zellen mit Extraktionspuffer II behandelt, nach Inkubation erhält man Fraktion 2 (F2)
iv: SAOS2-Zellen mit Extraktionspuffer III behandelt, nach Inkubation erhält man Fraktion 3 (F3)

### Beispiel 5: Proteinverteilung in Extrakten nach selektiver Detergenz-Extraktion von eukaryontischen Zellen nach dem Stand der Technik [1, 2, 3] im Vergleich zur der erfindungsgemäßen Methode.

Im erfindungsgemäßen Verfahren wurde in diesem Beispiel die Konzentration eines Alkalimetall-Salzes in Extraktionspuffer 3 im Vergleich zum Stand der Technik [1,2,3] erhöht und zusätzlich eine Nuklease eingesetzt. Zur Dokumenation der erzielten Verbesserung wurde Extraktionspuffer III mit variierenden Salzkonzentrationen (Stand der Technik, 15 mM NaCl + Benzonase®, 150 mM NaCl + Benzonase® und 350 mM NaCl + Benzonase®) angesetzt. Die Benzonase® (unspezifische Endonuklease) wurde bei allen Versuchen in gleicher Konzentration eingesetzt. Alle anderen Parameter entsprechen den im Stand der Technik angegebenen. Als biologisches Material wurden 4 mal ca. 80% konfluente SAOS 2-Zellen eingesetzt. Die entstandenen Proteinextrakte wurden mittels Proteinbestimmungsmethode, welche auf Lowry-Assay basiert, untersucht und ausgewertet.
Das erfindungsgemäße Verfahren deutet auf eine effizientere Extraktion des Zellkerns hin. Dies wird durch eine signifikante Erhöhung des Proteingehalts der nukleären Fraktion (F3) und eine Abnahme der Proteinmenge in der Cytoskelett-Fraktion (F4) deutlich sichtbar.

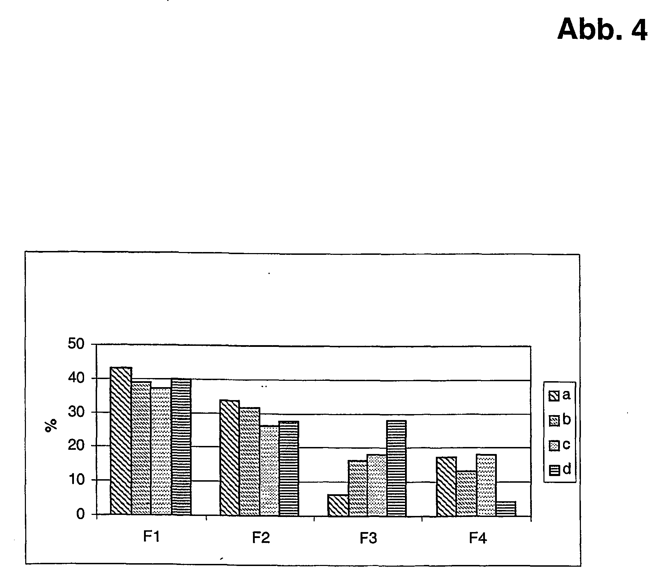

Die Abbildung 4 zeigt die Ergebnisse der Proteinbestimmung und deren Verteilung auf die vier Fraktionen.
Durch die Erhöhung der Salzkonzentration in Puffer lll kann im Zusammenhang mit der Wirkung von Benzonase® eine Umverteilung von Proteinen aus Fraktion 4 (F4) in Fraktion 3 (F3) erreicht werden. In den Fraktionen F1 und F2 wird erwartungsgemäß keine signifikante Veränderung beobachtet.
a) Stand der Technik
b) 15 mM NaCl in Puffer III + Benzonase (Stand der Technik + Benzonase)
c) 150 mM NaCl in Puffer III + Benzonase
d) 350 mM NaCl in Puffer III + Benzonase

### Beispiel 6: Untersuchungen zur Wirkung des erfindungsgemäßen Extraktionspuffers (Puffer III) mit und ohne Zusatz einer Nuklease

In den Proteinextrakten aus Beispiel 5 wurde ebenfalls die Verteilung von Markerproteinen für die entsprechenden Fraktionen mittels immunologischer Methoden nach der Auftrennung durch SDS-PAGE und Blotting auf eine PVDF-Membran untersucht.

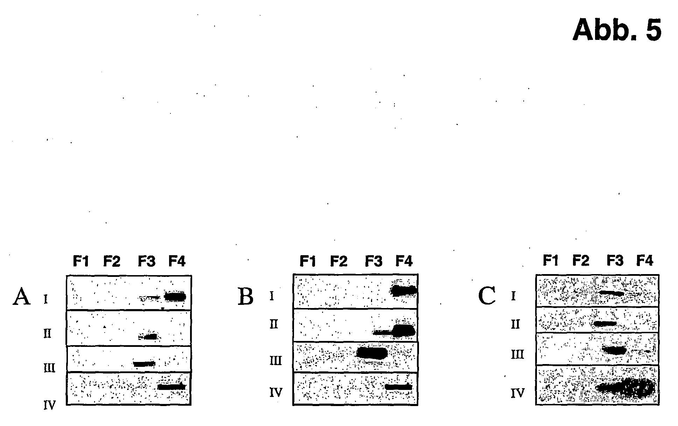

Die in Abbildung 5 dargestellte Analyse demonstriert die Verteilung von Transkriptionsfaktoren (am Beispiel c-jun Abbildung 5A) und DNA-assoziierten Proteinen (am Beispiel Histon 1 Abbildung 5B) in Fraktionen nach selektiver Detergenz-Extraktion in Abhängigkeit von der NaCl-Konzentration und der Einwirkung einer Nuklease. Die Integrität des Cytoskeletts wird nicht signifikant beeinträchtigt.
Um die Wirkung der Benzonase® zu testen, wurde in Gegenwart von 350 mM NaCl in Extraktionspuffer III, mit und ohne Nukleasewirkung in Fraktion 3, das Verhalten der Transkriptionsfaktoren und Histonproteine untersucht (Abbildung 5C). Der Einsatz der Nuklease bei gleich bleibender NaCl-Konzentration ermöglicht eine eindeutig erhöhte Wiederfindungsrate der DNA-assoziierten Histon-Proteine in der Fraktion 3, wohingegen die Transkriptionsfaktoren in Gegenwart von 350 mM NaCl ohne Nukleasewirkung extrahiert werden können.

Detaillierte Beschreibung der Abbildung 5:
**5A)** Die Wiederfindungsrate des Transkriptionsfaktors c-jun in Fraktion 3 ist abhängig von der NaCl-Konzentration in Puffer 3. Ab einer Konzentration von 150 mM NaCl in Puffer 3 kann c-jun in der Fraktion 3 detektiert werden (I: c-jun/15 mM NaCl; II: c-jun/150mM NaCl; III c-jun/350 NaCl). Keine dieser Bedingungen beeinträchtigt die Integrität des Cytoskeletts, was durch die ausschließliche Detektion von Cytokeratin in Fraktion 4 dokumentiert ist (IV: Cytokeratin/350 mM NaCl). Bei allen Experimenten wurde Benzonase^{®} eingesetzt. Bei Extraktion gemäß dem Protokoll des Standes der Technik [1, 2, 3] wird c-jun in der vierten Fraktion detektiert (I).
**5B)** Das Verhalten von Histon 1 bei unterschiedlichen Salzkorizentrationen in Puffer 3. Das DNA-assoziierte Protein Histon 1 wurde erst bei 350 mM NaCl in Puffer 3 und gleichzeitiger Nuklease-Wirkung in Fraktion 3 detektiert (I: Histon 1/15 mM NaCI; II: Histon 1/150mM NaCl; III: Histon 1/350 NaCl). Bei Extraktion gemäß dem Protokoll des Standes der Technik [1, 2, 3] wird Histon 1 in der vierten Fraktion gefunden (I).
**5C)** In Gegenwart von 350 mM NaCl in Extraktionspuffer 3 wird c-jun unabhängig von der Nukleasewirkung in Fraktion 3 detektiert. (I: c-jun/Puffer 3 mit Benzonase; II: c-jun/Puffer 3 ohne Benzonase). Histon 1 hingegen bedarf der gleichzeitigen Einwirkung von Nuklease, um zusammen mit c-jun in Fraktion 3 detektiert zu werden (III: Histone/Puffer 3 mit Benzonase; IV: Histone/Puffer 3 ohne Benzonase). Der Einsatz der Nuklease bei gleich bleibender NaCl-Konzentration ermöglicht eine eindeutig erhöhte Wiederfindungsrate DNA-assoziierter Proteine in der Fraktion 3.

### Beispiel 7: Morphologische Darstellung von Zell-Kompartimenten/ Organellen bei der erfindungsgemäßen Durchführung der selektiven Detergenz-Extraktion

Die Selektivität des erfindungsgemäßen Extraktionsverfahrens für die entsprechenden subzellulären Partialproteome wurde durch Fluoreszenzmikroskopie dokumentiert. Die erfindungsgemäße Proteinextraktion wurde mit einem Zellpräparat transfizierter COS-1-Zellen, ansonsten analog zu Beispiel 3, durchgeführt. Die Zellen wurden vor der Extraktion, d.h. nach dem Waschen (Bild I), und schrittweise nach dem jeweiligen Extraktionsschritt (Bild II-V) auf verfahrensbedingte morphologische Veränderungen fluoreszenzmikroskopisch untersucht.

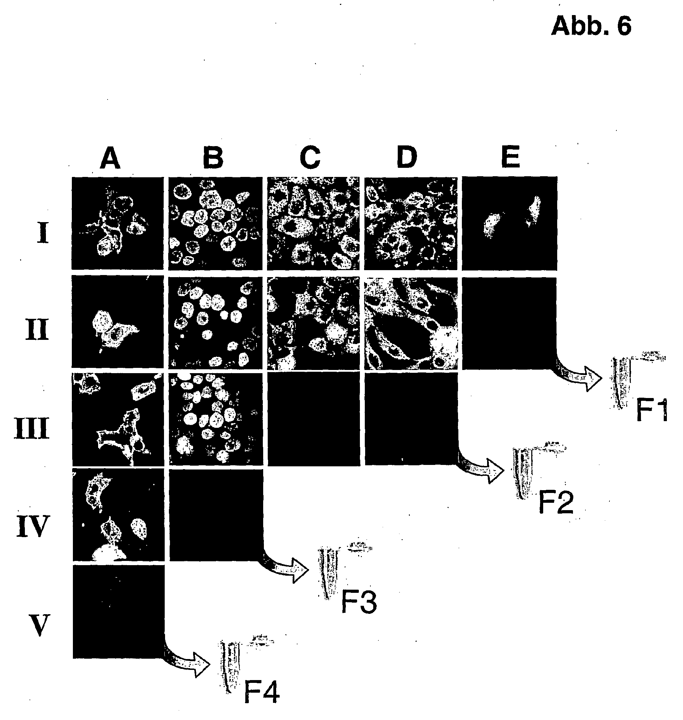

Abbildung 6 zeigt die subzellulären Kompartimente bzw. Organellen während der erfindungsgemäßen Extraktion am Beispiel von COS-1-Zellen. Das Waschen der Zellen zur Entfernung ansonsten störender Mediumbestandteile beeinträchtigt die Morphologie der Zellen nicht (Bild I). Nach der Behandlung der Zellen mit Extraktionspuffer I (Bild II), treten die cytosolischen Inhalte der Zellen aus, wobei die Integrität der Zellorganellen, der Zellkerne und des Cytoskeletts weitestgehend erhalten bleibt. Nach der Inkubation der Zellen mit dem Extraktionspuffer II (Bild III) werden die Membran/Organellen-Proteine extrahiert, wobei die Integrität der Zellkerne und des Cytoskeletts immer noch erhalten bleibt. Nach der Inkubation des restlichen Zellmaterials mit dem Extraktionspuffer III (erfindungsgemäßer Verfahrensschritt c) (Bild IV) wird die Fraktion der Proteine des Zellkerninneren extrahiert wobei die Integrität des Cytoskeletts weitestgehend erhalten bleibt. Das restliche Zellmaterial wird im Extraktionspuffer IV aufgenommen. Man erhält eine Fraktion, in der die Cytoskelettbestandteile enthalten sind.
F 1: Fraktion 1
F 2: Fraktion 2
F 3: Fraktion 3
F 4: Fraktion 4

Abbildung 6 demonstriert die Selektivität der erfindungsgemäßen Methode anhand der Visualisierung subzellulärer Kompartimente bzw. Organellen vom transfizierten COS-1-Zellen mittels kommerziell erhältlichen Fluoreszenzfarbstoffen: Phalicidin zum Anfärben des filamentösem Aktincytoskeletts, DAPI zur Visualisierung der Zellkerne, ER-Tracker™ zur Visualisierung der Endoplasmatisches Retikulum, MitoTracker zur Visualisierung der Mitochondrien (als Membran/Organellen-Marker) und lösliches, grün-fluoreszierendes Protein (GFP), welches von den transfizierten COS-1-Zellen exprimiert wird, und als Markerprotein für das Cytoplasma benutzt wird.

Beschreibung der Abb. 6:
A: Phallicidin, B: DAPI-Färbung, C: MitoTracker, D: ER-Tracker™, E: GFP
I: transfizierte COS-1-Zellen unbehandelt
II: transfizierte COS-1-Zellen mit Extraktionspuffer 1 behandelt, nach Inkubation erhält man Fraktion 1 (F1)
III: transfizierte COS-1-Zellen, nach Behandlung mit Extraktionspuffer I zusätzlich mit Extraktionspuffer II behandelt, nach Inkubation erhält man Fraktion 2 (F2)
IV: transfizierte COS-1-Zellen, nach Behandlung mit Extraktionspuffern I und II zusätzlich mit Extraktionspuffer III behandelt, nach Inkubation erhält man Fraktion 3 (F3)
V: transfizierte COS-1-Zellen, nach Behandlung mit Extraktionspuffern I bis III zusätzlich mit Extraktionspuffer IV behandelt, nach Inkubation erhält man Fraktion 4 (F4)

### Beispiel 8: Proteinmuster von Extrakten und topologische Zuordnung von subzellulären Markerproteinen nach selektiver Detergenzextraktion von eukaryontischen Zellen nach erfindungsgemäßen Extraktionsverfahren

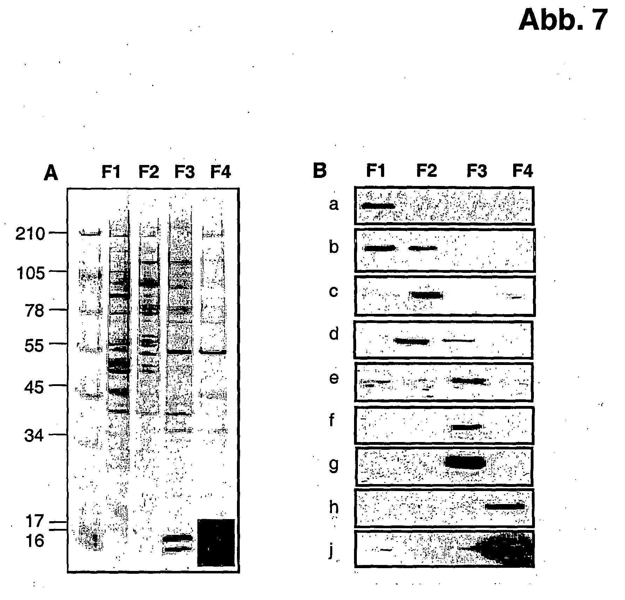

Die Proteinextrakte, die nach erfindungsgemäßer Extraktion von SAOS 2-Zellen gemäß Beispiel 3a und 3b gewonnen wurden; wurden mittels SDS-PAGE (10% Tricine-PAA-Gel) aufgetrennt. Die Proteine wurden anschließend mit Coomassie-Brilliant-Blue detektiert. Ein solches Ergebnis ist in Abbildung 7A zu sehen.
Zusätzlich wurden die Proteinextrakte nach der Trennung durch SDS-PAGE auf PVDF-Membranen geblottet und durch Immunoblot-Analyse gegen ausgesuchte Markerproteine untersucht. Die Ergebnisse sind in Abbildung 7B dargestellt.
In Übereinstimmung mit der Proteinmengenbestimmung in Beispiel 4 und den morphologischen Befunden in Beispiel 6 kann hier (7A und 7B) demonstriert werden, dass die effiziente Freisetzung der DNA-assoziierten Proteine des Zellkerninneren ermöglicht wird, ohne dabei die Integrität des Cytoskeletts zu beeinträchtigen. Nach erfindungsgemäßer Extraktion einer Zellpräparation gemäß Beispiel 3 wird somit eine eigenständige Fraktion der Proteine des Zellkerninneren erhalten, was somit erstmals die getrennte Betrachtung funktioneller Proteine von Zellkern und Cytoskelett ohne den Verlust anderer Partialproteome erlaubt.

Detaillierte Beschreibung der Abbildung 7:
7A) Nach selektiver Detergenz-Extraktion eukaryontischer Zellen anhand der erfindungsgemäßen Methode können eindeutig verschiedene Proteinmuster der eigenständigen subzellulären Fraktionen beobachtet werden. Hierbei bezeichnet F1 die Fraktion 1 mit den cytosolischen Proteinen. F2 steht für Fraktion 2 und stellt die Membran/Organellen Proteine dar. F3 (Fraktion 3) entspricht den Proteinen des Zellkerninneren. F4 (Fraktion 4) schließlich repräsentiert die Proteine des Cytoskeletts und der Kernmatrix. Im Vergleich zur Extraktion nach dem Stand der Technik [1, 2, 3] (Abb. 2) werden deutlich mehr Proteine in Fraktion 3 detektiert.
**7B) Verteilung von Markerproteinen auf subzelluläre Fraktionen nach selektiver Detergenz-Extraktion.**
   Nach selektiver Detergenz-Extraktion von eukaryontischen Zellen anhand der erfindungsgemäßen Methode ist im Gegensatz zum Stand der Technik (Abb. 3) eine eindeutige topologische Zuordnung von nukleären Markerproteinen möglich. Markerproteine des Cytosols und der Membran/Organellen werden in den entsprechenden Fraktionen detektiert (a-d). Lösliche Transkriptionsfaktoren (e und f) sowie das DNA-assoziierte Protein Histon 1 (g) werden eindeutig in einer Fraktion der Proteine des Zellkerninneren angereichert. Erst im letzten Schritt wird getrennt hiervon der hochabundante Cytoskelettmarker Cytokeratin (h und j) extrahiert.
   a: Calpain; b: Hitzeschock Protein 70 (HSP 70); c: Cadherin; d: Cytochrome
   P 450 reductase; e: c-fos; f: c-jun; g: Histone;
   h: Cytokeratin; j: Vimentin

### Beispiel 9: Proteinmuster von Extrakten und topologische Zuordnung von subzellulären Markerproteinen nach selektiver Detergenzextraktion von Darmkrebsgewebe nach erfindungsgemäßen Extraktionsverfahren

Ausgehend aus Darmkrebsgewebe wurden Zellcluster nach Standardverfahren [8] hergestellt und mit dem erfindungsgemäßem Extraktionsverfahren behandelt. Daraus resultierende Extrakte wurden mittels SDS-PAGE aufgetrennt, auf PVDF-Membranen transferiert und durch lmmunoblot-Analyse gegen ausgewählte Markerproteine untersucht. Die ausgewählten Markerproteine lauten wie folgt:
I = Calpain, II = Cytochrom p 450 reductase, lll = c-jun.
In dieser Abbildung bezeichnet F1 die Fraktion 1 mit den cytosolischen Proteinen. F2 steht für Fraktion 2 und stellt die Membran/Organellen Proteine dar. F3 (Fraktion 3) entspricht den Proteinen des Zellkerninneren. Das restliche Zellmaterial, welches in Extraktionspuffer IV aufgenommen werden kann, wurde in diesem Beispiel nicht untersucht.
Die Analyse der Immunoblots mit ausgewählten Markerproteinen bestätigt die Einsetzbarkeit der selektiven Detergenzextraktion nach erfindungsgemäßen Extraktionsverfahren mit Zellpräparaten von Gewebe, die nach Standardverfahren [8] hergestellt wurden.

### Beispiel 10: Untersuchung der dynamischen subzellulären Redistribution von NFkappaB, welcher an der Signaltransduktion beteiligt ist und nach zellulärer Stimulierung mit TNFalpha vom Cytosol in den Zellkern transloziert wird.

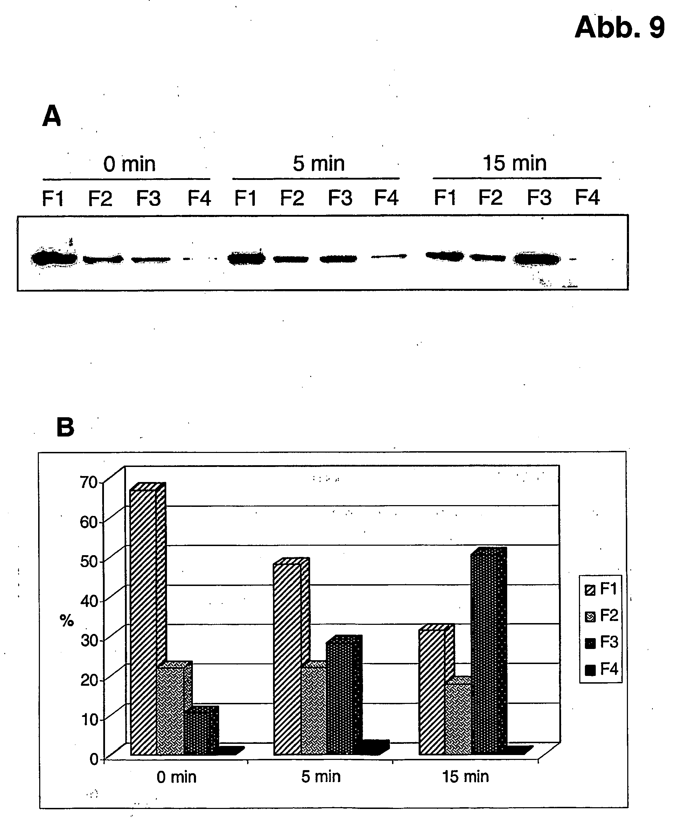

A431 Zellen wurden unterschiedlich lang (0, 5 und 15 Minuten) mit TNFα stimuliert und entsprechend dem erfindungsgemäßen Verfahren (siehe Beispiel 3) extrahiert. Die cytosolische Fraktion (F1), die Membran/Organell-Fraktion (F2), die nukleare Fraktion (Fraktion der Proteine des Zellkerninneren) (F3) wie auch die Cytoskelett-Fraktion (F4) wurden für den jeweiligen Stimulierungszeitpunkt mittels SDS-PAGE getrennt. Anschließend wurde ein Immunoblot mit einem spezifischen Antikörper gegen NFkappaB durchgeführt. Die Zeitverlaufsanalyse demonstriert eindeutig eine Umverteilung von NFkappaB- aus dem Cytosol in den Zellkern. Abbildung 9a zeigt die Ergebnisse der Immunoblot-Analyse. Die Intensität der Proteinbanden wurde densitometrisch bestimmt. Die gemessenen Daten wurden im Verhältnis zueinander graphisch dargestellt (siehe Abbildung 9b).

Das erfindungsgemäße Verfahren eignet sich, um die dynamischen subzellulären Redistribution von NFkappaB, welches an der Signaltransduktion beteiligt ist und nach zelluläre Stimulierung mit TNFalpha vom Cytosol im Zellkern transloziert wird, einfach und direkt mittels Immunoblot zu analysieren.

### Beispiel 11: Der Nachweis endogener Enzymaktivitäten nach selektiver Detergenzextraktion von eukaryontischen Zellen mit dem erfindungsgemäßen Extraktionsverfahren demonstriert sowohl Selektivität als auch Funktionalität representativer Enzyme in den Extrakten I, II und III.

Nach selektiver Detergenzextraktion von eukarontischen Zellen mittels der erfindungsgemäßen Methode wurden Enzymaktivitäten von repräsentative Markerenzyme untersucht als. Hierbei wurde die Aktivitäten für Calpain (a), als Markerenzym für das Cytoplasma, für alkalischen Phosphatase (b), als Markerenzym für Membran/Organellen, und für endogene zellulären RNAsen bestimmt (c). RNAsen sind Enzyme, welche in mehrere Formen innerhalb der Zelle vorkommen können und abhängig vom Zelltyp und spezifischen Faktoren mit verschiedenen subzellulären Kompartimenten assoziert sein können. Zum Schutz zellulärer RNA liegt RNase meist kompartimentalisiert vor. So wurde beispielsweise einerseits RNase LX [9] als auch mehrere RNase A-Subtypen mit Membran/Organellen assoziert [10] vorgefunden, RNase Hll anderseits ist im Zellkern lokalisiert [11].

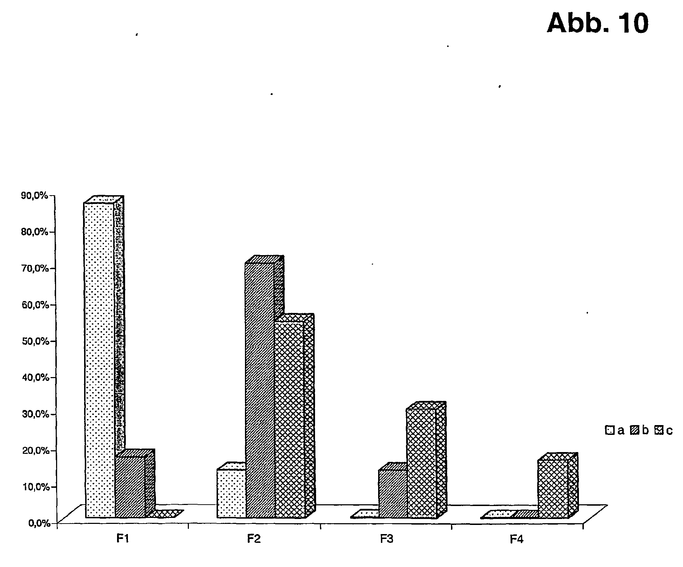

Für die Bestimmung der Enzymaktivität wurden Zellextrakte eingesetzt, welche erfindungsgemäß nach der Vorschrift in Beispiel 3 hergestellt wurden. Im Falle der Bestimmung der Calpain-Aktivität als Markerenzym für das Cytoplasma wurden hierzu Zellpräparationen humaner Hautkrebszellen (A 431) eingesetzt, für der Bestimmung der Aktivität der alkalischen Phosphatase als Markerenzym für Membran/Organellen (b) wurden Zellextrakte von SAOS 2-Zellen eingesetzt. Um zu dokumentieren, dass auch die Fraktion der Zellkernproteine nach der erfindungsgemäßen Extraktion von Zellpräparationen in nativem Zustand erhalten wird, wurde die endogene Aktivität der zellulären RNAsen bestimmt, welche auch im Zellkern vorkommen (siehe oben). Um es zu ermöglichen, die Aktivität dieser endogenen RNAsen zu bestimmen, wurden zusätzliche Zellextrakte von SAOS 2-Zellen hergestellt, wobei hierfür *keine* Nuklease im Extraktionspuffer III zugesetzt wurde
Die Ergebnisse sind in Abbildung 10 graphisch dargestellt, und demonstrieren die Selektivität der erfindungsgemäßen Methode zur Zellfraktionierung in Übereinstimmung mit den morphologischen Befunden (Abbildung 6) und den Ergebnissen nach der Immunoblot-Analyse(Abbildung 7). Etwa 90 % der bestimmten Aktivität des Calpains kann in der dem Cytoplasma zugeordneten Fraktion detektiert werden. Die Aktivität der alkalischen Phosphatase, welche als Membranmarker eingesetzt wurde, wird zu über 70 % in der Membrari/Organell-Fraktion detektiert. Die Aktivität zellulärer RNAsen verteilt sich nach der erfindungsgemäßen Extraktion ohne Nuklease Zusatz wie folgt: 54 % in der Membran/Organell-Fraktion, 30 % in der Fraktion der Zellkernproteine und 16 % in der Fraktion der CytoskelettProteine. In der Fraktion des Cytoplasmas konnte keine RNAse-Aktivität festgestellt werden. Die erfindungsgemäß eingesetzten Extraktionspuffer, besonders auch derjenige zur Herstellung des Partialproteoms der Proteine des Zellkerninneren, erhalten aktive, nicht denaturierte Proteine. Somit eignet sich das erfindungsgemäße Verfahren zur Analyse der subzellulären Verteilung von Enzymaktivitäten.

F1-F4 bezeichnen die Fraktionen 1-4. Die relative . Enzymaktivitäten für Calpain (a), alkalische Phosphatase (b) und RNAse (c) sind in dieser Abbildung graphisch dargestellt.

### Beispiel 12: Die Fraktion der Proteine des Zellkerninneren enthält funktionelle, hinsichtlich DNA-Bindung aktive Transkriptionsfaktoren.

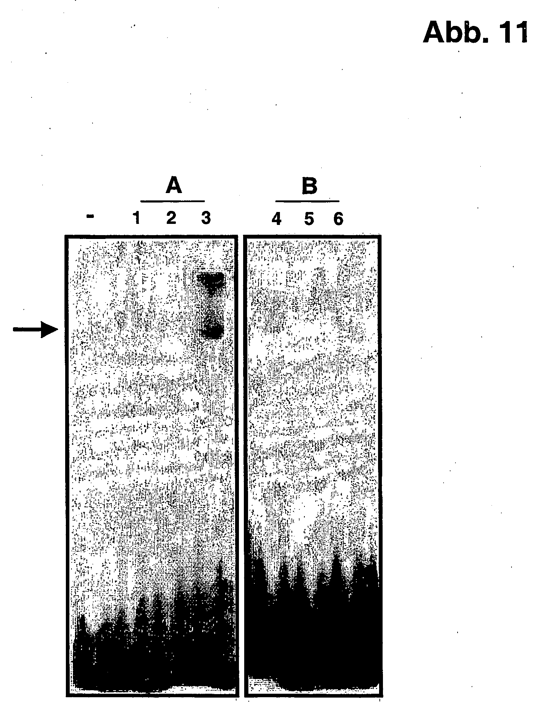

CHO-K1 Zellen wurden nach dem erfindungsgemäßen Extraktionsverfahren (gemäß Beispiel 3) extrahiert, jedoch ohne Benzonase^{®}-Zusatz im Extraktionspuffer III (Abbildung 11A). Ebenfalls wurde nach einem Standardverfahren für die spezielle Herstellung von nuklearen Extrakten [12] gewonnene Fraktion der Proteine des Zellkerninneren, die aktive Transkriptionsfaktoren enthält, als Positivkontrolle (Abbildung 11 B) eingesetzt. Anschließend wurden Proben der erhaltenen nuklearen Fraktionen mittels EMSA analysiert um die vorhandene Bindungsaktivität von Transkriptionsfaktoren an ein Oct1-Oligonukleotid zu bestimmen. Die Analyse demonstriert eindeutig, dass die nukleare Fraktion nach dem erfindungsgemäßen Verfahren funktionelle, hinsichtlich DNA-Bindung aktive Transkriptionsfaktoren enthält.
Dies ist eine beispielhafte Demonstration der breiten Anwendbarkeit des erfindungsgemäßen Verfahrens.

Beschreibung der Abbildung 11:
1. mit erfindungsgemäß erhaltener Fraktion der Proteine des Zellkerninneren
2. mit erfindungsgemäß erhaltener Fraktion der Proteine des Zellkerninneren + 100x nicht radioaktiv markierter Oct1-Probe
3. mit erfindungsgemäß erhaltener Fraktion der Proteine des Zellkerninneren+ 100x nicht radioaktiv markierter SP1-Probe
4. nach Standardverfahren erhaltene Fraktion der Proteine des Zellkerninneren
5. nach Standardverfahren erhaltene Fraktion der Proteine des Zellkerninneren + 100x nicht radioaktiv markiertem Oct1-Probe
6. nach Standardverfahren erhaltene Fraktion der Proteine des Zellkerninneren+ 100x nicht radioaktiv markiertem SP1-Probe

## Patentansprüche

1. Verfahren zur sequentiellen Herstellung von Partialproteomen aus dem Gesamtproteom einer Zellpräparation, **gekennzeichnet durch** folgende Verfahrensschritte:
a) Bereitstellen einer Probe enthaltend eine Zellpräparation;
b) Extraktion der cytosolischen Proteine und der Membran/Organellen-Proteine aus der in Schritt a) bereitgestellten Probe, wobei eine Zellkernpräparation zurückbleibt;
c) Extraktion der Proteine des Zellkerninneren aus der in Schritt b) erhaltenen Zellkernpräparation **durch** Behandlung mit einem Extraktionspuffer mit einem pH-Wert zwischen 6,5 und 8, der zumindest die folgenden Bestandteile enthält:
- insgesamt 0,1 bis 7 Massenprozent eines oder mehrerer nicht ionischer Detergentien
- insgesamt 0,05 bis 3 Massenprozent eines oder mehrerer Cholsäurederivate
- eines oder mehrere Salze aus der Gruppe der Alkalimetall-und/oder Ammoniumsalze in einer Gesamtkonzentration zwischen 75 und 500 mMol/l,
wobei man ein Partialproteom erhält, das angereichert ist an Proteinen des Zellkerninneren, wie DNA-bindenden Proteinen, Nichthiston Proteinen (z.B. HMGs und Transkriptionsfaktoren), RNA-bindenden Proteinen und Skelett-asoziierten Proteinen.

2. Verfahren nach Anspruch 1, **dadurch gekennzeichnet, dass** der in Schritt c) eingesetzte Extraktionspuffer zusätzlich eine Nuklease enthält.

3. Verfahren nach einem der Ansprüche 1 oder 2, **dadurch gekennzeichnet, dass** der in Schritt c) eingesetzte Extraktionspuffer Polyoxyethylensorbitanmonopalmitat als nicht-ionisches Detergenz, Deoxycholat als Cholsäurederivat und NaCl als Alkalimetallsalz enthält.

4. Verfahren nach einem oder mehreren der Ansprüche 1 bis 3, **dadurch gekennzeichnet, dass** die Extraktion der cytosolischen Proteine und der Membran/Organellen-Proteine in Schritt b) erfolgt durch:
i) Extraktion der cytosolischen Proteine aus der in Schritt a) bereitgestellten Probe durch selektive Permeabilisierung der Plasmamembran durch Behandlung mit einem Saponin, Behandlung mit Streptolysin-O, enzymatische Einwirkung mittels z.B. Lipase, Einwirkung mechanischer Kräfte wie z,B. Elektroporation, Einfrieren/Auftauen, Filter-Ripping oder einer Kombination der genannten Möglichkeiten;
ii) Extraktion der Membran/Organellen-Proteine aus dem in Schritt i) nach der Extraktion verbliebenen Teil der Probe durch Behandlung mit nicht-ionischen oder zwitter-ionischen Detergenzien.

5. Verfahren nach einem oder mehreren der Ansprüche 1 bis 4, **dadurch gekennzeichnet, dass** in einem zusätzlichen Verfahrensschritt d) aus dem in Schritt c) verbliebenen Extraktionsrückstand die Proteine des Detergenz-resistenten Cytoskeletts und der Kernmatrix als weiteres Partial-Proteom extrahiert werden.

6. Kit für die Proteinextraktion zumindest enthaltend einen Extraktionspuffer mit einem pH-Wert zwischen 6,5 und 8, der zumindest die folgenden Bestandteile enthält:
- insgesamt 0,1 bis 7 Massenprozent eines oder mehrerer nichtionischer Detergentien
- insgesamt 0,05 bis 3 Massenprozent eines oder mehrerer Cholsäurederivate
- eines oder mehrere Salze aus der Gruppe der Alkalimetall-und/oder Ammoniumsalze in einer Gesamtkonzentration zwischen 75 und 500 mMol/l,
sowie einen Puffer zur Extraktion der cytosolischen Proteine, einen Puffer zur Extraktion der Membran/Organellen-Proteine und einen Puffer zur Extraktion der Proteine des Detergenz-resistenten Cytoskeletts und der Kernmatrix.

7. Kit nach Anspruch 6, zusätzlich enthaltend eine Nuklease.

## Claims

1. Method for the sequential production of partial proteomes from the complete proteome of a cell preparation, **characterised by** the following method steps:
a) provision of a sample containing a cell preparation;
b) extraction of the cytosolic proteins and the membrane/organelle proteins from the sample provided in step a), leaving a cell nucleus preparation;
c) extraction of the cell nucleus interior proteins from the cell nucleus preparation obtained in step b) by treatment with an extraction buffer having a pH of between 6.5 and 8 which comprises at least the following constituents:
- in total 0.1 to 7 per cent by weight of one or more nonionic detergents
- in total 0.05 to 3 per cent by weight of one or more cholic acid derivatives
- one or more salts from the group of the alkali metal and/or ammonium salts in a total concentration of between 75 and 500 mmol/l;
giving a partial proteome which is enriched with cell nucleus interior proteins, such as DNA-binding proteins, non-histone proteins (for example HMGs and transcription factors), RNA-binding proteins and skeleton-associated proteins.

2. Method according to Claim 1, **characterised in that** the extraction buffer employed in step c) additionally comprises a nuclease.

3. Method according to one of Claims 1 and 2, **characterised in that** the extraction buffer employed in step c) comprises polyoxyethylene sorbitan monopalmitate as nonionic detergent, deoxycholate as cholic acid derivative and NaCl as alkali metal salt.

4. Method according to one or more of Claims 1 to 3, **characterised in that** the extraction of the cytosolic proteins and the membrane/organelle proteins in step b) is carried out by:
i) extraction of the cytosolic proteins from the sample provided in step a) by selective permeabilisation of the plasma membrane by treatment with a saponin, treatment with streptolysine-O, enzymatic action by means of, for exam[ple, lipase, the action of mechanical forces, such as, for example, electroporation, freezing/thawing, filter ripping or a combination of the said possibilities;
ii) extraction of the membrane/organelle proteins from the part of the sample remaining after the extraction in step i) by treatment with nonionic or zwitterionic detergents.

5. Method according to one or more of Claims 1 to 4, **characterised in that** the detergent-resistant cytoskeleton proteins and the nuclear matrix proteins are, in an additional method step d), extracted as a further partial proteome from the extraction residue remaining in step c).

6. Protein extraction kit at least containing an extraction buffer having a pH of between 6.5 and 8 which comprises at least the following constituents:
- in total 0.1 to 7 per cent by weight of one or more nonionic detergents
- in total 0.05 to 3 per cent by weight of one or more cholic acid derivatives
- one or more salts from the group of the alkali metal and/or ammonium salts in a total concentration of between 75 and 500 mmol/l,
and a buffer for extraction of the cytosolic proteins, a buffer for extraction of the membrane/organelle proteins and a buffer for extraction of the detergent-resistant cytoskeleton proteins and the nuclear matrix proteins.

7. Kit according to Claim 6, additionally containing a nuclease.

## Revendications

1. Procédé pour la production séquentielle de protéomes partiels à partir du protéome complet d'une préparation de cellules, **caractérisé par** les étapes de procédé qui suivent:
a) fourniture d'un échantillon contenant une préparation de cellules;
b) extraction des protéines cytosoliques et des protéines de membrane/d'organite à partir de l'échantillon fourni au niveau de l'étape a), en laissant une préparation de noyaux de cellules;
c) extraction des protéines intérieures de noyaux de cellules à partir de la préparation de noyaux de cellules obtenue au niveau de l'étape b) par traitement avec un tampon d'extraction présentant un pH entre 6,5 et 8, lequel comprend au moins les constituants qui suivent:
- au total de 0,1 à 7 pour cent en poids d'un ou de plusieurs détergents non ioniques
- au total de 0,05 à 3 pour cent en poids d'un ou de plusieurs dérivés d'acide cholique
- un ou plusieurs sels pris parmi le groupe des sels de métal alcalin et/ou d'ammonium selon une concentration totale entre 75 et 500 mmol/l;
ce qui donne un protéome partiel qui est enrichi avec des protéines intérieures de noyaux de cellules, telles que des protéines de liaison ADN, des protéines non histones (par exemple des HMG et des facteurs de transcription), des protéines de liaison ARN et des protéines associées au squelette.

2. Procédé selon la revendication 1, **caractérisé en ce que** le tampon d'extraction utilisé au niveau de l'étape c) comprend additionnellement une nucléase.

3. Procédé selon l'une des revendications 1 et 2, **caractérisé en ce que** le tampon d'extraction utilisé au niveau de l'étape c) comprend du monopalmitate de polyoxyéthylène sorbitan en tant que détergent non ionique, du désoxycholate en tant que dérivé d'acide cholique et du NaCl en tant que sel de métal alcalin.

4. Procédé selon une ou plusieurs des revendications 1 à 3, **caractérisé en ce que** l'extraction des protéines cytosoliques et des protéines de membrane/d'organite au niveau de l'étape b) est mise en oeuvre par:
i) extraction des protéines cytosoliques à partir de l'échantillon fourni au niveau de l'étape a) par perméabilisation sélective de la membrane de plasma par traitement avec une saponine, par traitement avec streptolysine-O, par action enzymatique au moyen, par exemple, d'une lipase, par action de forces mécaniques, tel que, par exemple, une électroporation, une congélation/décongélation, une désagrégation par filtre ou une combinaison desdites possibilités;
ii) extraction des protéines de membrane/d'organite à partir de la partie de l'échantillon qui reste après l'extraction au niveau de l'étape i) par traitement avec des détergents non ioniques ou zwitterioniques.

5. Procédé selon une ou plusieurs des revendications 1 à 4, **caractérisé en ce que** les protéines de cytosquelette résistant aux détergents et les protéines de matrice nucléaires sont, selon une étape de procédé additionnelle d), extraites en tant qu'autre protéome partiel à partir du résidu d'extraction qui reste au niveau de l'étape c).

6. Kit d'extraction de protéine contenant au moins un tampon d'extraction présentant un pH entre 6,5 et 8, lequel comprend au moins les constituants qui suivent:
- au total de 0,1 à 7 pour cent en poids d'un ou de plusieurs détergents non ioniques
- au total de 0,05 à 3 pour cent en poids d'un ou de plusieurs dérivés d'acide cholique
- un ou plusieurs sels pris parmi le groupe des sels de métal alcalin et/ou d'ammonium selon une concentration totale entre 75 et 500 mmol/l,
et un tampon pour l'extraction des protéines cytosoliques, un tampon pour l'extraction des protéines de membrane/d'organite et un tampon pour l'extraction des protéines de cytosquelette résistant aux détergents et des protéines de matrice nucléaires.

7. Kit selon la revendication 6, contenant additionnellement une nucléase.
